(19)

Europäisches Patentamt

European Patent Office

Office européen des brevets

(11) **EP 3 928 774 B1**

(12) **EUROPEAN PATENT SPECIFICATION**

(45) Date of publication and mention of the grant of the patent:
**13.12.2023   Bulletin 2023/50**

(51) International Patent Classification (IPC):
***A61K 31/10*** *(2006.01)*      ***A61P 43/00*** *(2006.01)*

(21) Application number: **20182483.6**

(52) Cooperative Patent Classification (CPC):
**A61K 31/10**

(22) Date of filing: **26.06.2020**

(54) **DIMETHYL SULFONE FOR TREATMENT OF CHRONICALLY HEAT STRESSED POULTRY**

DIMETHYLSULFON ZUR BEHANDLUNG VON CHRONISCH WÄRMEBELASTETEM GEFLÜGEL

SULFONE DIMÉTHYLIQUE POUR LE TRAITEMENT DE LA VOLAILLE CHRONIQUEMENT STRESSÉE PAR LA CHALEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.12.2021   Bulletin 2021/52**

(73) Proprietor: **Evonik Operations GmbH**
**45128 Essen (DE)**

(72) Inventors:
• **Hess, Vincent**
  **63456 Hanau (DE)**
• **De Paula Dorigam, Juliano Cesar**
  **63456 Hanau (DE)**

• **Michiels, Joris**
  **8432 Leffinge (BE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
• **M. S. ABDUL RASHEED ET AL: "Dietary methylsulfonylmethane supplementation and oxidative stress in broiler chickens", POULTRY SCIENCE, vol. 99, no. 2, 22 January 2020 (2020-01-22), pages 914-925, XP55754363, Oxford ISSN: 0032-5791, DOI: 10.1016/j.psj.2019.12.010**

EP 3 928 774 B1

**Description**

The present invention relates to dimethyl sulfone and a composition for use in the treatment by oral administration of heat stress in chronically heat stressed poultry.

Animals are divided into two groups: cold-blooded (heterothermic) and warm-blooded (homeothermic). Birds are homeothermic, as they have the ability to maintain a stable internal body temperature regardless of external influence. However, their thermoregulatory mechanisms are efficient only in thermoneutral zones. General features of thermoregulation in birds are similar to those of other homeothermic animals. However, in contrast to homeothermic species, birds do not have sweat glands. Further, birds use feathers and fat insulation, which is a good insulation in cold weather but inhibits heat loss in hot weather. Therefore, birds have difficulties in dissipating heat through the feather coverage. This makes them more susceptible to develop a stress under high temperatures. Specifically, birds have air sacs, that allow the inhaled air, which is usually cooler than the body temperature, to reach deep into the abdominal capacity so that when the bird exhales, heat is removed from the body. Further, birds use a panting mechanism (also referred to as gular flutter) during hot weather to evaporate water from its throat, thus reducing body temperature. Panting is extremely effective in cooling birds. In addition, birds, as endotherms, can regulate body temperature using heat generated in their bodies. Heat is produced in the body of birds as a result of metabolism and muscular activity. The concentrations of enzymes, vitamins, and hormones; physical activities; oxygen consumption; ambient temperature; and circadian rhythms affect the amount of heat production in the bodies of chickens. To maintains body temperature and avoid overheating, excess heat is dissipated to the surrounding environment through cellular conduction and vascular convection. Inability of heat dissipation leads to metabolic disorders and in extreme situation to the death of the individual.

Therefore, high ambient temperatures can be devastating to commercial animals. Coupled with high humidity they can have an even more harmful effect. Heat stress interferes with animal comfort and suppresses productive performance, reproductive performance, economic traits, and welfare of poultry. Heat stress can be chronic or acute. Sudden and short periods of extremely high ambient temperatures and humidity can result in acute heat stress. An extended period of elevated ambient temperatures along with increased humidity results in chronic heat stress. Birds are 'heat stressed' if they have difficulty achieving a balance between body heat production and body heat loss. Heat stress can occur at all ages and in all types of poultry. The temperature comfort zone of birds depends on their age. Older birds are more sensitive to high temperatures. For example, a temperature of 21°C is the ideal temperature for poultry in the grower period. Nevertheless, in principle one can generalize that birds feel comfortable at ca. 21 to 24 °C (75 °F) and function normally up to ca. 27 °C (80 °F). Above 27 °C (up to ca. 30 °C), feed consumption drops, while water intake increases. Feed conversion ratios (FCRs) and weight gain decrease in broiler birds, and egg production decreases in layer and breeder flocks. At 30 to 32 °C (86 to 95 °F), a significant decline in egg production and egg-shell quality is observed. In layers, the FCR based on egg mass and the FCR per dozen eggs increase as ambient temperature increase. When the temperature exceeds ca. 35 to 37 °C (96 to 100 °F), birds attempt to reduce body heat through severe gular fluttering; however, temperatures in this range result in high degree of mortality. Additionally, marked depression, nervous behaviors and symptoms such as trembling, staggering, and convulsions can also be seen.

At a temperature of 35 °C (95 ° F) and a relative humidity of 40%, birds can dissipate 80% of their total heat through evaporative heat loss (gular fluttering), but at a temperature of 35 °C (95 ° F) and a relative humidity of 50%, heat loss dissipation is reduced to only 50%. At a temperature of 35 °C and a relative humidity of 100%, heat cannot be lost from the body anymore, resulting in severe stress, shock and high mortality. A temperature of ca. 38°C (101 °F) is lethal; at this temperature, mortality risk increases, and emergency measures are needed.

Feed consumption and Feed Conversion Factor are affected by high temperatures. Specifically, body weight gain, egg production, in particular egg size, egg quality and hatchability, as well as fertility are decreased in breeder flocks exposed to heat stress.

The article "Dietary methylsulfonylmethane supplementation and oxidative stress in broiler chickens" (M. S. Abdul Rasheed et al., Poultry Science, vol. 99, no. 2, 22 January 2020, page 914-925) discussed the effects of dimethyl sulfone on oxidative markers in chickens stressed with oxidized oil. However, a significant change in outcomes was not shown in this article. Further, this article does not disclose any reduction in heat stress in broiler chickens.

Commercial poultry farming is a very profitable business; however, any type of heat stress drastically reduces the profitability of poultry farming. Solutions for the protection from heat stress require multifactorial strategies that may involve housing, genetics, thermal conditioning and feeding and nutrition. A multitude of different strategies was designed to mitigate heat stress in poultry. In short, these strategies can be divided into the three groups mechanical techniques, specifically housing and thermal conditioning; nutritional strategies, involving restricted feeding and watering strategies, use of phytochemicals such as lycopene, anthocyanins, gamma-glutamylethylamide, adjusting the dietary electrolyte balance, specifically with potassium bicarbonate, potassium chloride, sodium bicarbonate, sodium chloride, and ammonium chloride in water and/or feed, supplementing of vitamins, in particular vitamin C and E, supplementing of virginiamycin, betaine, and prebiotics/probiotics, reducing crude protein content using feed grade amino acids, increase of fats by 3 to 5% in feed at the cost of carbohydrates without changing the metabolizable energy, increase of digestible amino acids by about 5 to 10% than normally used; and other management, such as activities at the farm, specifically, do not disturb the birds for transportation, debeaking, dubbing, etc. during peak heat period; early heat conditioning, e.g. induce heat resistance, and litter management, e.g. litter should be kept dry and not be much wet. However, none of the strategies has proven to be successful for treatment of heat stress in poultry. In particular, none of the strategies has proven to be successful for reducing mortality in poultry being exposed to heat stress. Accordingly, there was still a need for an approach for the successful reduction of mortality in poultry being exposed to heat stress, specifically chronic heat stress.

It was found that this need is met by the oral administration of dimethyl sulfone to poultry being exposed to chronic heat stress.

One object of the present invention is therefore dimethyl sulfone for use in the treatment of heat stress in poultry by oral administration, wherein the dimethyl sulfone is administered to chronically heat stressed poultry.

Poultry receiving dimethyl sulfone (DMS) administration showed an increased heat tolerance and thus, a reduced mortality compared to poultry not receiving DMS administration. Specifically, DMS administration to poultry led not only to a reduced panting and thus a reduced loss of moisture, but also to a reduced rectal temperature. Specifically, DMS administration to poultry supports poultry to adapt their metabolism better to heat stress. For example, DMS appeared to promote the antioxidant function and redox homeostasis in poultry. This results in a lower oxidative stress or higher antioxidant capacity in heat stressed poultry. At the same time, DMS administration to poultry diet did not negatively affect the general performance of the poultry nor the meat quality; specifically, there was no significant white striping or wooden breast observable in the carcass.

The term poultry is used in the context of the present invention to denote any kind of domesticated bird, captive raised for its utility. Examples for poultry are domestic fowls, including chickens, turkeys, geese, quails and ducks, raised to produce meat or eggs. Preferably, the term poultry refers to chickens in the context of the present invention.

The term heat stress is used in the context of the present invention to determine the exposure to elevated ambient temperature. Heat stress can be chronic or acute. The term chronic heat stress is used in the context to the present invention to determine an extended period of elevated ambient temperature. In contrast, acute heat stress is understood in the context of the present invention to determine a sudden and short period of extremely high ambient temperatures. In principle, the dimethyl sulfone according to the present invention is not subject to a limitation regarding a chronic or an acute heat stress. The term elevated ambient temperature is used in the context of the present invention and in particular in the content of the term chronic heat stress to determine a temperature which is above the comfortable temperature of poultry. As already said above, the temperature comfort zone of birds depends on their age. Older birds are more sensitive to high temperatures. Typically, birds feel comfortable at ca. 21 to 24 °C (70 to 75 °F) and function normally up to ca. 27 °C (80 °F). However, above 27 °C (up to ca. 30 °C), feed consumption drops, while water intake increases. Feed conversion ratios (FCRs) and weight gain decrease in broiler birds, and egg production decreases in layer and breeder flocks. At temperatures from 30 to 32 °C (86 to 95 °F), a significant decline in egg production and egg-shell quality is observed. In layers, the FCR based on egg mass and the FCR per dozen eggs increase as ambient temperature increase. When the temperature exceeds ca. 35 to 37 °C (96 to 100 °F), birds attempt to reduce body heat through severe gular fluttering; however, temperatures in this range result in some degree of mortality. Preferably, a temperature of more than 27 °C and in particular, a temperature of at least 30 °C is an elevated ambient temperature in the context of the present invention. In particular, a temperature in the range from 30 to 40 °C is an elevated temperature.

In an embodiment of the dimethyl sulfone according to the present invention the poultry is exposed to a temperature of more than 27 °C, preferably to a temperature of at least 30°C.

The term an extended period or an extended period of elevated ambient temperature is used in the context of the present invention to determine a period of at least 5 hours a day. Preferably, the extended prior or extended period of elevated ambient temperature denotes a period of 5 to 24 hours a day, in particular at least 5, 6, 7, 8, 9, 10, 11, 12, 13, 14, 15, 16, 17, 18, 19, 20, 21, 22, 23 or even up to 24 hours a day.

In one embodiment of the dimethyl sulfone according to the present invention the poultry is exposed to a temperature of more than 27 °C, preferably to a temperature of at least 30 °C, for at least 5 hours a day.

Often it is the case that the elevated temperature to the poultry is exposed follows a cyclic course: The temperature has a minimum value at night, then steadily increases over the day until it reaches a maximum, and from this maximum the temperature decreases again over night until the minimum value. If the described temperature course extends over several days or weeks, it is also called a chronic cycle heat stress in the context of the present invention.

In a preferred embodiment of the dimethyl sulfone according to the present invention, the chronic heat stress is chronic cyclic heat stress.

In many cases, specifically in southern countries, in particular in subtropical, tropical and in general in those countries near the equator, an elevated temperature is coupled with a higher relative humidity. The term higher relative humidity is used in the context of the present invention to denote a relative humidity of at least 40% on average. Preferably, the relative humidity ranges from 40 to 70% on average, in the context of the present invention, in particular from 50 to 60%, on average.

In another embodiment of the dimethyl sulfone according to the present invention the poultry is exposed to a relative humidity of at least 40% on average.

The lifetime of captive-raised birds, i.e. poultry, can be divided into the three phases of starter, grower and finisher phase. For example, the whole lifetime of chicken amounts to 39 days, of which the days (d) from d-0 to d-10 are called the starter phase, the days from d-10 to d-21 are called the grower phase and the days from d-21 to d-39 are called the finisher phase. In principle, the dimethyl sulfone according to the present invention being administered to chronically heat stress poultry is not limited to any specific phase or period in the lifetime of poultry. Therefore, the dimethyl sulfone can be administered to chronically heat stressed poultry at any conceivable time point in or during any of the phases, i.e. in or during the starter, grower and/or finisher phase. Notwithstanding, it is preferred to administer the dimethyl sulfone to chronically heat stressed poultry in or during the finisher phase. The term in with respect to a phase is used in the context of the present invention to denote selected time points, such as hours, days, or weeks, which are not necessarily a continuous period. In contrast, the term during with respect to a phase is used in the context of the present invention to denote to continuous period of hours, days or weeks.

In a further embodiment of the dimethyl sulfone according to the present invention the dimethyl sulfone is administered to poultry in or during the starter, grower and/or finisher phase.

Preferably, the dimethyl sulfone according to the present invention is administered to poultry starting from the beginning of starter phase until slaughter or from the beginning of grower phase until slaughter.

Notwithstanding, it appeared that DMS supplemented birds were better prepared in the previous feeding period, hereby referring to improved feed conversion ratio in the following feeding period, to cope with the upcoming heat stress. Specifically, it appeared that providing birds with a DMS supplemented in the grower period supported the poultry to cope better with the upcoming heat stress in the following finishing period.

In a preferred embodiment of the dimethyl sulfone according to the present invention the dimethyl sulfone is administered to poultry in the finishing period.

Preferably, the dimethyl sulfone according to the present invention is administered to poultry together and/or accompanied by a low protein diet and/or a diet with low amount of digestible lysine. Preferably, the said diet nevertheless has an ideal amino acid profile in order to avoid or at significantly reduce any negative impact on the body weight gain of poultry.

The dimethyl sulfone according to the present invention being administered to chronically heat stressed poultry can be part of any matrix, preferably a liquid and/or a solid matrix. For example, the dimethyl sulfone can be administered to chronically heat stressed poultry through their drinking water, their diet or combination of both. It was found that a concentration of only 0.05 wt.-% of the dimethyl sulfone, based on the total weight of the liquid and/or solid matrix, through which the dimethyl sulfone is administered, already has a beneficial effect on chronically heat stressed poultry.

Preferably, the dimethyl sulfone is administered to the poultry in a concentration of at least 0.05 wt.-%, based on the total weight of a liquid and/or solid matrix.

It is further preferred that the dimethyl sulfone is administered to the poultry in a concentration from 0.05 to 0.5 wt.-%, from 0.05 to 0.4 wt.-% or from 0.5 to 0.3 wt.-%, based on the total weight of a liquid and/or solid matrix.

Another object of the present invention is therefore also a composition comprising dimethyl sulfone for use in the treatment of heat stress in poultry by oral administration, comprising at least 0.05 wt.-% of dimethyl sulfone, based on the total weight of the composition, wherein the composition is administered to chronically heat stressed poultry.

In an embodiment the composition according to the present invention comprises dimethyl sulfone in a concentration from 0.05 to 0.5 wt.-%, based on the total weight of the composition.

Preferably, the composition comprises dimethyl sulfone in a concentration from 0.05 to 0.4 wt.-% or from 0.5 to 0.3 wt.-%, based on the total weight of a liquid and/or solid matrix.

Chronical heat stress is characterized by extended periods of elevated temperatures. As explained above in detail, a temperature of more than 27 °C, preferably a temperature of at least 30 °C, preferably in the range from 30 to 40 °C, is an elevated temperature in the context of the present invention. Further, an extended period of elevated temperature is understood to mean a period of at least 5 hours a day, preferably, a period of 5 to 24 hours a day, in particular at least t 5, 6, 7, 8, 9, 10, 11, 12, 13, 14, 15, 16, 17, 18, 19, 20, 21, 22, 23 or even up to 24 hours a day.

In a further embodiment of the composition according to the present invention the poultry is exposed to a temperature of more than 27 °C, preferably to a temperature of at least 30 °C.

In one embodiment of the composition according to the present invention the poultry is exposed to a temperature of more than 27 °C, preferably to a temperature of at least 30 °C, for at least 5 hours a day.

In a preferred embodiment of the composition according to the present invention, the heat stress is chronic cyclic heat stress.

In another embodiment of the composition according to the present invention the poultry is exposed to a relative humidity of at least 40% on average. Preferably, the relative humidity ranges from 40 to 70% on average, in the context of the present invention, in particular from 50 to 60%, on average.

In a further embodiment of the composition according to the present invention the composition is administered to poultry in or during the starter, grower and/or finisher phase.

Preferably, the composition is a liquid and/or a solid matrix.

When the composition according to the present invention is a solid matrix, it is preferred that said composition is a diet for poultry. Preferably, said diet is a starter, grower and/or finisher diet for poultry, in particular, a finisher diet.

When the composition according to the present invention is a diet for poultry, it is further preferred that is a low protein diet and/or a diet with a low amount of digestible lysine. Preferably, the diet nevertheless contains an ideal amino acid profile in order to avoid or at least significantly reduce a negative effect on the body weight gain of poultry.

When the composition according to the present invention is a liquid matrix, it is preferred that said composition is the drinking water for the poultry, supplemented with at least 0.05 wt.-% of dimethyl sulfone, based on the total weight of the drinking water.

When the environmental temperature rises, the birds will drink more water. This will also increase the quantity of liquid excreta in the pens and thus, will also increase the moisture in the litter. However, a wet litter has a poor quality and lead to food footpad health. Therefore, a wet litter is considered to have a negative impact on bird welfare as well. It was found that the administration of dimethyl sulfone according to the present invention and/or of a dimethyl sulfone comprising composition according to the present invention helps to reduce the quantity of liquid excreta in the pens and thus, reduces the overall moisture in the litter.

A further object of the present invention is therefore also dimethyl sulfone and/or a dimethyl sulfone comprising composition for use in reducing moisture, wherein the dimethyl sulfone according to the present invention and/or the dimethyl sulfone comprising composition according to the present invention is administered to chronically heat stressed poultry.

The present invention is further illustrated by the following figures and examples.

**Description of the figures:**

Fig. 1     shows the course of the relative humidity (upper line, right Y-axis) and of the temperature (lower line, left Y-axis) from d-0 until the end of the experiment.

Fig. 2     shows the course of the relative humidity (upper line, right Y-axis) and of the temperature (lower line, left Y-axis) from d-24 until the end of the experiment.

Fig. 3     is a scatterplot representation of the ADG for period from d-10 to d-21 per treatment with mean $\pm$ deviation.

Fig. 4     is a scatterplot representation of the F.G for the period from d-10 to d-21 per treatment with mean $\pm$ deviation.

Fig. 5     shows the progress of the body weight per treatment, and for male Ross 308 Performance Objectives 2019 and as projected for finisher phase.

Fig. 6     shows the feed intake per bird and per day during periods of 6 hours of heat stress (lower 3 lines) and during the rest of the day (upper 3 lines) in the period from d-24 till d-39 for each treatment.

Fig. 7     shows the percentage of total feed intake and the percentage of water intake being consumed during hours of heat stress in the period from d-24 till d-39 (6 h per day) for each treatment for all pens together.

Fig. 8     shows the mortality cases per day and treatment in the period from d-24 to d-39.

Fig. 9     is similar as Fig. 8 but excluding pen 24 (T2) having 5 mortality cases in the period from d-24 to d-39; cases of mortality in this period were 14, 10 and 8 for T1, T2 and T2, respectively.

Fig. 10    shows the percent survival of birds in the period of heat stress (d-24 to d-39; days in heat stress 1 and 16 correspond to d-24 and d-39, respectively) using Kaplan-Meier representation based on data of Fig. 9. Log-ranked test for trend, $p$=0.159; suggesting no statistical difference in survival curves. Fig. 10 is similar as Fig. 3 but excluding pen 24 (T2) habing 5 mortality cases in period from d-24 to d-39. Cases of mortality in this period were 14, 10 and 8 for T1, T2 and T3, respectively.

Fig. 11    shows mortality+culling cases per day and treatment in the period from d-24 to d-39 (cullings are dotted blocks). Cases of mortality+culling in this period were 16, 17 and 8 for T1, T2 and T, respectively.

Fig. 12    shows mortality+culling cases per day and treatment in the period from d-24 to d-39 (cullings are dotted blocks). Similar as Fig. 11 but excluding pen 24 (T2) having 5 mortality cases in the period from d-24 to d-39. Cases of mortality+culling in this period were 16, 12 and 8 for T1, T2 and T, respectively.

Fig. 13    shows the percent survival of birds in the period of heat stress (d-24 to d-39; days in heat stress 1 and 16 correspond to d-24 and d-39, respectively) using Kaplan-Meier representation based on data of Fig. 12. Log-ranked test for trend, $p$=0.087; suggesting no statistical difference in survival curves. Fig. 13 is similar as Fig. 3 but excluding pen 24 (T2) having 5 mortality cases in period from d-24 to d-39. Cases of mortality in this period were 14, 10 and 8 for T1, T2 and T3, respectively.

Fig. 14    is a boxplot + Tukey whiskers representation of rectal temperature of birds during heat stress per treatment.

Fig. 15    is a boxplot + Tukey whiskers representation of rectal temperature of birds sampled at d-23, d-25, and d-39.

Fig. 16    shows the concentration of T3, malondialdehyde and glutathione as well as the T3/T4 ratio in the blood of male broilers subjected to chronic cyclic heat stress sampled at d-25 by time after starting heat stress.

Fig. 17    show the relation between malondialdehyde and glutathione in the blood of male broilers subjected to chronic cyclic heat stress sampled at d-39.

Fig. 18    shows the effect of DMS administration on litter moisture.

**Experiments**

1. Materials and methods

1.1 Experimental design

The experimental design consisted of one control group (T1) and two other groups receiving the control diet supplemented with increasing levels of DMS (dimethyl sulfone), specifically 0.1% DMS (T2) and 0.2% DMS (T3). Treatments were replicated in 12 pens each in a completely randomized block design. Block refers to physical allocation in the experimental facility. All the animal trial was realized in compliance with the Ethics Committee of Faculty of Veterinary Medicine (Ghent University, EC2018-71).

1.2 Birds and housing

The experiment was conducted at the experimental facilities of the Department of Animal Sciences and Aquatic Ecology of Ghent University, located in Melle, Belgium. A total of 792 male broiler chickens (Ross 308, Aviagen Group) were provided by Vervaeke-Belavi hatchery (Tielt, Belgium). Larger treatment effects are expected with males, as they are more sensitive to heat stress (HS) (Cahaner and Leenstra, 1992). The experiment consisted in three rearing phases: starter (from d-0 to d-10), grower (from d-10 to d-21) and finisher (from d-21 to d-39). The one-day old chicks were weighted and equally spread among the pens according to their body weight (BW). Pens were randomly allocated to the treatment groups. The initial average animal BW was 41.3 $\pm$ 0.31 g at the beginning of the experiment. The newly selected chicks were housed in a single climate-controlled room, which contained 36 pens of 22 animals each. Each pen had a dimension of 0.80 x 1.70 m, and had concrete floor covered with wood shavings. The room was equipped with liner drinkers (two nipples per pen), and each pen had one tube-type metallic feeder. Temperature in starter and grower phase was set to decrease linearly from 34°C, at d-0 until a basal temperature of 22°C, at d-24. The light of the room was turned on 23h in period d-0 to d-7, and then turned on at 4:00 and switched off at 22:00 during the rest of the experiment (18L:6D).

1.3 Heat stress protocol

The finisher period started at d-21, and the chronic cyclic heat stress (HS) protocol was applied from d-24 until d-39. Starting from d-24, the temperature was increased from 22°C to 34°C for 6 hours per day (from 9:00 to 15:00) with relative air humidity (RH) between 52 and 58%. From 8:00 to 9:00, the temperature was increased from 22°C to 34°C, and from 15:00 to 16:00, the temperature was decreased from 34°C to 22°C. The rest of the day the temperature was 22°C. The room was equipped with heaters with thermostats, and air circulators to enable the desired temperature and RH to be maintained, providing sufficient ventilation and air conditions. RH was adjusted with manual spraying of water on stable walls, periodically. The temperature and the RH were recorded at 10-min intervals with a dry bulb thermo-hygrometer (Escort RH iLog, Escort Verification Technologies Inc., Buchanan, United States).

1.4 Dietary treatments

Wheat and soybean meal-based feeds were formulated to meet the nutrient requirements of broiler chickens, according to Ross308 recommendations or slightly lower to avoid excessive mortality in the finisher phase. Diets did not contain coccidiostats, nor synthetic antioxidants, nor emulsifiers. The final formulation was approved by the commissioner. Ingredient and nutrient composition of all diets are given in Table 1 and 2. The test substance dimethyl sulfone was added on top at 0.1 and 0.2% in T2 and T3 in all rearing phases, respectively. Diets were processed by Research Dietary Services (RDS, Wijk bij Duurstede, The Netherlands) and delivered to Ghent University. All the diets were provided as pellets during all rearing phases. The chickens were fed *ad libitum* and had free access to water during the whole experiment.

1.5 Performance and health records

For all performance indices, the pen is the experimental unit. Total feed intake (FI) was recorded for each rearing period. In addition, FI during HS period was recorded every day, weighing the feeders before (at 8:00) and after (at 16:00) HS every day. That allows the calculation of the relative FI during period with HS relative to the total FI for each day during HS (d-24 to d-39). The total body weight (BW) of each pen was recorded at d-0, d-10, d-21 and d-39, and divided by the number of chickens in the pen at that day to have the average BW for each pen. Average daily gain (ADG, equation 1), average daily feed intake (ADFI, equation 2), the feed-to-gain ratio (F:G, equation 3) and the mortality rate (MR, equation 4) were calculated using the formulas below :

$$(1) \quad ADG_P = \frac{BW_{last\,day} - BW_{first\,day}}{last\,day - first\,day}$$

where $ADG_P$ is the ADG at a given rearing period (g/d), $BW_{first\,day}$ is the average BW of the pen at the first day of the studied rearing period (g) with *first day* = 0, 10 or 21 for the starter, grower and finisher period, respectively, $BW_{last\,day}$ is the average BW of the pen at the last day of the studied rearing period (g) with *last day* = 10, 21 or 39 for the starter, grower and finisher period, respectively.

$$(2) \quad ADFI_p = \frac{TFI_p}{AD_p}$$

where $ADFI_p$ is the ADFI per bird (g/d) at a given rearing period, $TFI_p$ is the total FI of the pen during the given rearing period (g), and $AD_p$ is the number of animal-days during this rearing period (day).

$$(3) \quad F{:}G_p = \frac{ADFI_p \times AD_p}{TBW_{last\,day} + BW_{dead} - TBW_{first\,day}}$$

where $F{:}G_p$ is the feed:gain ratio (g/g) at a given rearing period, $ADFI_p$ is the ADFI per bird (g/d), $AD_p$ is the number of animal-days during this rearing period (d), $TBW_{first\,day}$ is the total BW of the pen at the first day of the studied rearing period (g) with *first day* = 0, 10 or 21 for the starter, grower and finisher period, respectively, $TBW_{last\,day}$ is the total BW of the pen at the last day of the studied rearing period (g), with *last day* = 10, 21 or 39 for the starter, grower and finisher period respectively, and $BW_{dead}$ is the total BW of the dead chickens during the studied period, including sampled birds in case (g).

All the dead birds were collected, and their weights were recorded every day. The MR was calculated for each rearing period using the following ratio:

$$(4) \quad MR_p = \frac{\#birds_{first\,day} - \#birds_{last\,day}}{\#birds_{first\,day}} \times 100$$

where $MR_p$ is the MR at a given period (%), #birds$_{first\,day}$ is the number of animals present in the pen the first day of the period, with *first day* = 0, 10 or 21 for the starter, grower and finisher period, respectively, #birds$_{last\ day}$ is the number of animals present in the pen the last day of the period with *last day* = 10, 21 or 39 for the starter, grower and finisher period, respectively. The sampled chickens at d-23 and d-25 were excluded from the respective calculation.

Finally, the European production efficiency factor (EPEF, equation 5) was calculated as follows:

$$(5)\quad EPEF = \frac{(100 - MR) \times BW_{d-39}}{39 \times F{:}G} \times 100$$

where *EPEF* is the European Production Efficiency Factor, MR is the MR for the total period (%), $BW_{d-39}$ is the average BW at d-39 (kg) and *F:G* is the F:G (g/g) for the total period.

1.6 Sampling and physiological measurements

Rectal temperature and panting frequency (video analyses) (for 2 selected birds per pen on 3 days: d-24, d-31 and d-38, measured after 4h of heat stress that day)

Sampling of one bird per pen (with weight close to average weight of the pen) on d-23, d-25 and on d-39 (on d-25 and d-39, sampling starts after >3h of heat stress that day; 108 birds in total)

- malondialdehyde, HSP70 (*Chicken Heat Shock Protein 70, Hsp-70 ELISA Kit*) *Cusabio Code CSB-E11196Ch*), HSP90 (*Chicken Heat Shock Protein 90, HSP-90 ELISA Kit; Cusabio Code CSB-E12873C*), GPx, SOD in plasma; GSH/GSSG in erythrocytes; and T3, T4 and nitric oxide in serum

- Breast muscle and liver: malondialdehyde, AOX (GPx, SOD), GSH/GSSG

Sampling of two birds per pen (with weight close to average weight of the pen) on d-40 after overnight fasting (no heat stress that day)

- Carcass yield, breast percentage, thigh percentage, drumstick percentage and abdominal fat

- Carcass yield: with skin, bled, without internal organs, without feet, without head, without neck, without abdominal fat; relative to bird body weight

  Breast percentage: skinless, boneless; relative to bird body weight
  Thigh percentage: with skin, with bone; relative to bird body weight
  Drumstick percentage: with skin, with bone; relative to bird body weight
  Abdominal fat percentage: abdominal fat; relative to bird body weight

- Breast meat quality

  - Scoring of breast myopathies (wooden breast and white striping)

    Wooden breast: normal, 0; mild, 1 and severe, 2
    White striations: normal, 0; mild, 1, and severe, 2

  - pH and colour, both at time 0h and 24h (after sampling at time 0h, breasts are stored at 4°C until 24h)
  - Drip loss, determined for periods 0-24 h and for period 0-72 h (after sampling at time 0h, breasts are stored at 4°C until 72 h, and weight without exuded water at 24 h and 72 h)

  - Press loss, determined at 24 h (after sampling at time 0h, breasts are stored at 4°C until 24 h, prior to determine press loss)
  - Oxidative stability (TBARS after simulated retail display)

1.7 Statistical analysis

All data were checked for anomalies and outliers (see description of results). Next, data for each dependent variable were tested for normal distribution (Kolmogorov-Smirnov) and homogeneity of variances (Levene's test) across treatments. It appeared that data for mortality and for mortality+culling were not normally distributed. Hence, data for this endpoint was evaluated with the non-parametric Kruskal-Wallis test, whereas other endpoints were tested with GLM procedures, unless otherwise stated.

Data were analyzed with the following statistical model:

$$Y_j = \mu + D_j + \varepsilon_j$$

with $Y_j$ the mean value of treatment $j$ (T1, T2 and T3), $\mu_i$ is the overall mean, $Dj$ is the fixed effect of treatment j, and $\varepsilon_j$ is the error term. Block was further included as random factor if significant. In case, day (e.g. $FI_{HS}$, rectal temperature) was included as within-subject factor (repeated measure). Orthogonal contrasts were applied to test for linear and quadratic effects upon incremental levels of DMS supplementation in the diet. For all analyses, pen was considered the experimental unit. Means are given as estimated marginal means (least square means). Means were separated using the Tukey post-hoc test. Differences were considered significant when $p<0.05$ and as a tendency for significance when $p<0.1$.

For wooden breast and white striations statistical evaluation was done by the Chi-square test. Differences were considered significant when $p<0.05$ and as a tendency for significance when $p<0.1$.

For physiological measurements, time of sampling (representing the number of minutes between the beginning of heat stress and the precise moment of sampling of the bird that day), if appropriate, was included in the model as a covariable if significant as well. Orthogonal contrasts were applied to test for linear and quadratic effects upon incremental levels of DMS supplementation in the diet. Means are given as estimated marginal means (least square means). Means were separated using the Tukey post-hoc test. Differences were considered significant when $p<0.05$ and as a trend for significance when $p<0.1$.

**Table 1. Composition of wheat-soybean based basal diets for the starter (d-0 to 10), grower (d-10 to 21) and finisher (d-21 to 39) phase.**

| Item | Starter (d0-10) | Grower (d10-21) | Finisher (d21-39) |
|---|---|---|---|
| wheat batch A7855 | 59.75 | 67.32 | 69.01 |
| soybean meal batch A7845 | 23.45 | 15.79 | 13.11 |
| toasted soybeans batch A7833 | 10.00 | 10.00 | 10.00 |
| lard | 1.000 | 2.000 | 3.000 |
| soybean oil | 0.868 | 0.443 | 0.731 |
| monocalciumphosphate | 1.260 | 1.102 | 0.966 |
| limestone | 1.655 | 1.536 | 1.455 |
| salt | 0.232 | 0.181 | 0.203 |
| sodiumbicarbonate | 0.250 | 0.280 | 0.251 |
| premix article 252 (*) | 0.500 | 0.500 | 0.500 |
| choline CHL.50S | 0.100 | 0.100 | 0.050 |
| natuphos 5000AL (500 FTU) | 0.010 | 0.010 | 0.010 |
| L-lysine HCL | 0.329 | 0.308 | 0.313 |
| DL-methionine | 0.333 | 0.240 | 0.218 |
| L-threonine | 0.165 | 0.131 | 0.127 |
| L-valine | 0.088 | 0.049 | 0.046 |
| xylanase + glucanase | 0.010 | 0.010 | 0.010 |
| **Calculated nutrient content** | | | |
| dry matter [%] | 89.4 | 89.4 | 89.0 |
| ME poultry [kcal] | 3000 | 3100 | 3200 |
| crude protein [%] | 23.1 | 20.3 | 19.2 |

(continued)

| Calculated nutrient content | | | |
|---|---|---|---|
| ether extract [%] | 5.81 | 6.39 | 7.69 |
| crude ash [%] | 6.32 | 5.68 | 5.35 |
| starch [%] | 35.8 | 40.3 | 41.2 |
| calcium [%] | 0.96 | 0.87 | 0.81 |
| P dig. [%] | 0.48 | 0.44 | 0.41 |
| Na+K-Cl (meq/100g) [meq] | 25.0 | 22.0 | 20.5 |
| LYS [%] | 1.38 | 1.17 | 1.10 |
| dig LYS [%] | 1.24 | 1.05 | 0.99 |
| MET + CYS [%] | 1.03 | 0.87 | 0.83 |
| dig M+C (**)[%] | 0.93 | 0.79 | 0.74 |
| dig M+C / dig LYS [%] | 75 | 75 | 75 |
| THR [%] | 0.96 | 0.81 | 0.76 |
| dig THR [%] | 0.83 | 0.70 | 0.66 |
| dig THR / dig LYS [%] | 67 | 67 | 67 |
| TRP [%] | 0.30 | 0.26 | 0.24 |
| dig TRP [%] | 0.26 | 0.22 | 0.21 |
| dig TRP / dig LYS [%] | 21 | 21 | 21 |
| VAL [%] | 1.12 | 0.95 | 0.89 |
| dig VAL [%] | 0.99 | 0.84 | 0.79 |
| dig VAL / dig LYS [%] | 80 | 80 | 80 |
| ARG [%] | 1.47 | 1.25 | 1.17 |
| dig ARG [%] | 1.31 | 1.11 | 1.03 |
| dig ARG / dig LYS [%] | 106 | 105 | 104 |
| ILE [%] | 0.93 | 0.80 | 0.75 |
| dig ILE [%] | 0.82 | 0.71 | 0.66 |
| dig ILE / dig LYS [%] | 67 | 68 | 67 |
| LEU [%] | 1.63 | 1.41 | 1.33 |
| dig LEU [%] | 1.44 | 1.25 | 1.17 |
| dig LEU / dig LYS [%] | 116 | 119 | 119 |

(*) Premix Artikel 252 providing per kg of diet: vitamin A (retinyl acetate), 10000 IU; vitamin D3 (cholecalciferol), 2500 IU; vitamin E (dl-$\alpha$-tocopherol acetate), 50 mg; vitamin K3 (menadione), 1.5 mg; vitamin B1 (thiamine), 2.0 mg; vitamine B2 (riboflavin), 7.5 mg; niacin, 35 mg; D-pantothenic acid, 12 mg; vitamin B6 (pyridoxine-HCl), 3.5 mg; vitamin B12 (cyanocobalamine), 20 $\mu$g; folic acid, 1.0 mg; biotin, 0.2 mg; choline chloride, 460 mg; Fe ($FeSO_4.H_2O$), 80 mg; Cu ($CuSO_4.5H_2O$), 12 mg; Zn (ZnO), 60 mg; Mn (MnO), 85; I ($Ca(IO_3)_2$), 0.8 mg; Co ($Co_2CO_3(OH)_2$), 0.77 mg; Se ($Na_2O_3Se$), 0.15 mg.

(**) Dig M/Dig M+C is 66, 63 and 62% in starter, grower and finisher diet, respectively Dig = digestible, ME = metabolisable energy

2. Results

2.1 Diet formulation and analysis

Diets were properly prepared as pellets and delivered to the experimental facility. Samples were analysed for proximate components and amino acids by Evonik Nutrition & Care GmbH (Table 2). Regarding starter diets it can be concluded that supplemental levels of LYS, MET, THR, and VAL were in line with feed formulation, and that all nutrients across treatments were very similar. Further, analysed crude protein, ether extract and ash appeared slightly lower than anticipated. Taking into account an uncertainty of 3% for all amino acids, all analysed values for amino acids show consistency with formulated values. Congruence for metabolizable energy (ME) could not be evaluated as different MEs were used. Similar observations can be made for the grower diets. Thus, again crude protein was lower (between 0.3 and 0.5%) as formulated. Finally, finisher diets showed again no difference across treatments. The discrepancy in crude protein in T2 *versus* T1 and T3 cannot be confirmed by differences in amino acids levels, as amino acid contents of all finisher diets are very identical. Nonetheless, crude protein was again lower than formulated, similar to somewhat lower ether extract and ash.

**Table 2. Analysed composition (in %) of corn-soybean based basal diets for starter, grower and experimental finisher diets (d-21 to 39)[1,2].**

| Item | Starter (d0-d10) | | | Grower (d10-d21) | | | Finisher (d21-d39) | | |
|---|---|---|---|---|---|---|---|---|---|
| | T1 | T2 | T3 | T1 | T2 | T3 | T1 | T2 | T3 |
| dry matter (NIR) | 89.5 | 89.8 | 89.9 | 89.4 | 89.8 | 90.1 | 89.4 | 89.4 | 89.7 |
| AME (NIR) [kcal/kg] | 2885 | 2915 | 2898 | 2973 | 2984 | 2958 | 3039 | 3051 | 3054 |
| ether extract (NIR) | 5.6 | 5.6 | 5.5 | 6.3 | 6.2 | 6.0 | 7.2 | 7.1 | 7.1 |
| ash (NIR) | 5.8 | 5.6 | 5.6 | 5.1 | 5.0 | 5.0 | 4.6 | 4.8 | 4.7 |
| starch (NIR) | 36.9 | 37.5 | 37.2 | 40.4 | 40.5 | 40.4 | 41.8 | 42.3 | 41.7 |
| P (NIR) | 6.5 | 6.1 | 6.1 | 5.5 | 5.4 | 5.1 | 4.7 | 4.9 | 4.7 |
| crude protein | 22.8 | 22.7 | 22.7 | 20.0 | 19.8 | 19.8 | 18.7 | 18.3 | 18.8 |
| LYS | 1.40 | 1.39 | 1.39 | 1.18 | 1.18 | 1.17 | 1.10 | 1.10 | 1.10 |
| MET | 0.65 | 0.66 | 0.65 | 0.53 | 0.52 | 0.52 | 0.48 | 0.48 | 0.48 |
| CYS | 0.38 | 0.38 | 0.35 | 0.34 | 0.34 | 0.34 | 0.33 | 0.33 | 0.33 |
| MET+CYS | 1.02 | 1.03 | 1.00 | 0.86 | 0.86 | 0.86 | 0.81 | 0.81 | 0.81 |
| THR | 0.94 | 0.94 | 0.95 | 0.81 | 0.80 | 0.80 | 0.74 | 0.74 | 0.75 |
| Val | 1.09 | 1.09 | 1.09 | 0.92 | 0.92 | 0.92 | 0.86 | 0.87 | 0.87 |
| ARG | 1.44 | 1.43 | 1.45 | 1.22 | 1.23 | 1.23 | 1.13 | 1.14 | 1.13 |
| ILE | 0.92 | 0.92 | 0.91 | 0.78 | 0.79 | 0.78 | 0.73 | 0.73 | 0.73 |
| LEU | 1.60 | 1.58 | 1.59 | 1.38 | 1.38 | 1.38 | 1.29 | 1.29 | 1.29 |
| LYS supplemental | 0.28 | 0.28 | 0.28 | 0.26 | 0.25 | 0.25 | 0.26 | 0.26 | 0.25 |
| MET supplemental | 0.33 | 0.34 | 0.34 | 0.24 | 0.24 | 0.24 | 0.22 | 0.22 | 0.22 |
| THR supplemental | 0.16 | 0.17 | 0.17 | 0.14 | 0.14 | 0.14 | 0.13 | 0.13 | 0.13 |

(continued)

| Item | Starter (d0-d10) | | | Grower (d10-d21) | | | Finisher (d21-d39) | | |
|---|---|---|---|---|---|---|---|---|---|
| | T1 | T2 | T3 | T1 | T2 | T3 | T1 | T2 | T3 |
| VAL supplemental | 0.09 | 0.09 | 0.09 | 0.05 | 0.05 | 0.05 | 0.05 | 0.05 | 0.05 |
| [1] T1: control diet, T2: control diet + 0.1% DMS, and T3: control diet + 0.2% DMS<br>[2] Nutrients were analysed by wet chemistry and given in %, unless otherwise stated. | | | | | | | | | |

2.2 Room temperature and relative humidity during the study

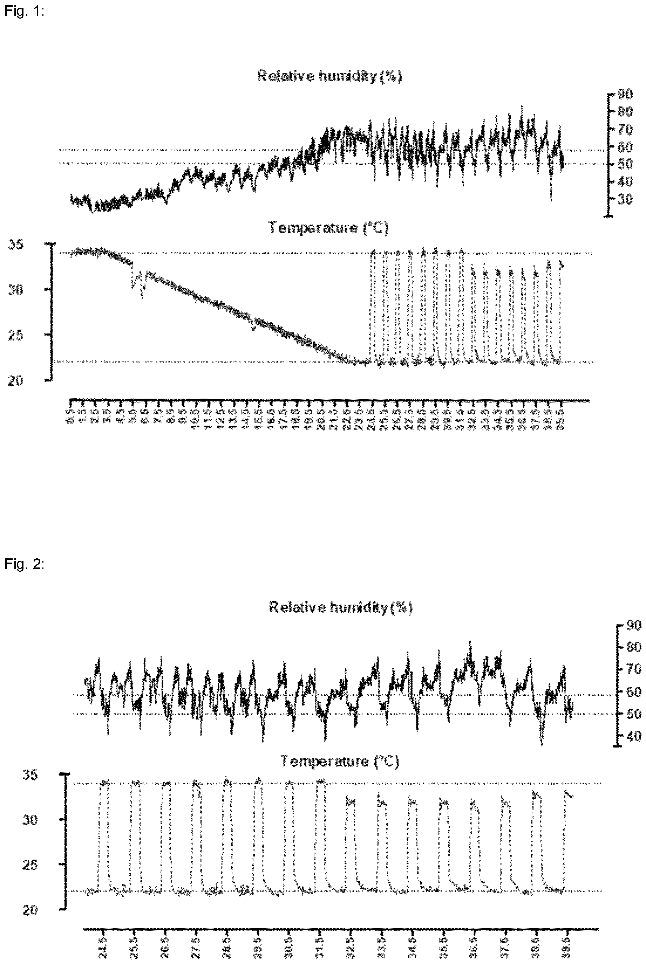

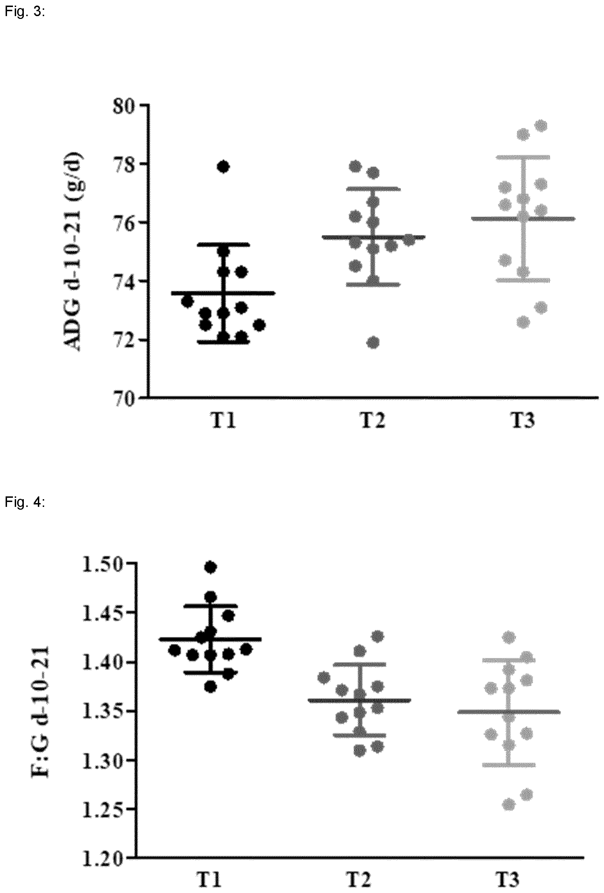

Temperature in starter and grower phase was set to decrease linearly until d-24, with final temperature of 22°C (Fig. 1). On d-5, infra-red lamps were shut down which caused a 3°C drop in temperature, but temperature remained acceptable for these aged birds, and was restored soon after. RH started low, but gradually increased towards the beginning of the finisher phase, when diurnal variations became larger due to HS protocol. Humidity was not controlled during starter and grower phases, and might depend on age of birds, temperature, outside humidity (rain, snow), ventilation rate and capacity, litter quality etc. Starting from d-24, the HS protocol was applied successfully (Fig. 1 and 2). However, 7, 5 and 5 mortality cases, respectively, were observed on d-28, d-29 and d-31 (Fig. 3). In order to prevent substantial bias of data due to excessive mortality it was decided to step down the heat stress protocol a little by having 32°C during heat stress period from d-32 to -37, which was again increased to 33°C for days 38 and 39. Fig. 3 shows that mortality decreased upon decreasing temperature during HS. During the period of HS, RH showed a clear diurnal pattern. Upon increasing stable temperature to 34°C to initiate HS, humidity tends to drop. However, target RH of 50-58% was maintained by regular nebulization of water, if necessary. During the rest of the day, RH showed big fluctuations, and reached occasionally >80% during night-time on some days.

Typically, upon initiating the HS protocol, birds tend to reduce movement, stop ingestion of feed, followed by immobility, spreading wings, increasing ground contact and ultimately severe panting. Effects on water intake are ambiguous; birds might increase water consumption due to evaporative and urinary losses, however, water consumption associated with feed intake is reduced. During the rest of the day, birds resumed normal behavior and (compensatory) feed intake.

2.3 Animal performances

Birds were found healthy throughout the study and performed altogether very well, with final body weights exceeding Male Ross 308 Performance Objectives 2019. Various endpoints showed that pen 1 (T2) and pen 2 (T1) (edge of stable) did not experience heat stress in period d-24-39 as other pens did; i.e. no or minimal panting was observed, feed intake during heat stress relative to total intake per day was higher and close to 33.3% which is equal to the ratio of duration of heat stress per day (6 h) to total period with light (18 h). Hence, it was concluded not to include pen 1 and pen 2 in statistical evaluation for any endpoints in finisher period and total period. Further, pen 24 (T2) showed excessive mortality in finisher period, i.e. 5 mortality cases thus equalling to 22.7%, which made calculations for performance indices biased. Hence, it was concluded not to include pen 24 in statistical evaluation for any endpoints in finisher period and total period. This is also highlighted in Fig. 3-6. Table 3 summarizes performance data of broilers for all rearing phases. In the starter period, no significant effects were found for either endpoint. Body weight at the end of starter period was around 360 g. In contrast, in grower period, final body weight and ADG showed linear increases with higher inclusion of DMS in the diet (P<0.05). ADG in this period was 2.6 and 3.4% higher for T2 and T3, respectively, as compared to control (T1) (both P<0.05), further illustrated by Fig. 3. This was explained by a remarkable improvement of feed conversion ratio. F:G was reduced by 6 and 7 points for T2 and T3, respectively, as compared to T1 (both P<0.05). Fig. 4 shows a scatterplot representation of F:G for this period. The finisher period encompassed two subperiods: d-21 to d-24 was without heat stress protocol, whereas in period d-24 to d-39 the heat stress protocol prevailed, reducing potential growth of birds. Surprisingly, none of the performance indicators was affected by treatment. Nonetheless, a numerical reduction of mortality by supplementing DMS could clearly be observed, mortality accounted for 6.3, 4.7 and 3.1% in T1, T2 and T3, respectively. From these data it is concluded that supplementing DMS increased heat tolerance of the animals and thus reduced mortality. A mortality of 6.3% is commonly seen when applying the chronic cyclic heat stress model in the used facility, but reductions as seen for T2 and T3 are rarely found. This emphasizes the importance of the numerical reductions here. In line with finisher phase, overall performance was not influenced by diet, but again numerical differences in mortality deserves full attention. The fact that DMS improved performance in the grower phase and not in the finisher might be related by the sharp reduction in formulated digestible LYS in grower diets as compared to starter diets (1.05 vs. 1.24%), which might have been far too low when consulting Ross 308 nutrient recommendations. The subsequent reduction in finisher diets was small (0.99 vs. 1.05%). Likely, DMS is more effective in low protein / low digestible LYS (whilst maintaining the ideal amino acid profile) diets.

**Table 3. Dietary effect on BW, ADG, ADFI, F:G, mortality and EPEF in male broilers subjected to chronic cyclic heat stress in the finisher phase[123] (n=10-12, i.e. 10-12 pen replicates per treatment).**

| Item | T1 | T2 | T3 | SEM[4] | P | | |
|---|---|---|---|---|---|---|---|
| | | | | | Model | Linear | Quadratic |
| **Starter (d0-d10)** | | | | | | | |
| Initial BW [g] | 41.3 | 41.4 | 41.2 | 0.05 | 0.176 | 0.289 | 0.123 |
| Final BW [g] | 362 | 359 | 357 | 1.2 | 0.258 | 0.105 | 0.834 |
| ADG [g/d] | 32.0 | 31.7 | 31.6 | 0.12 | 0.266 | 0.111 | 0.783 |
| ADFI [g/d] | 32.5 | 32.5 | 32.3 | 0.12 | 0.665 | 0.444 | 0.638 |
| F:G | 1.02 | 1.03 | 1.03 | 0.001 | 0.150 | **0.057** | 0.716 |
| Mortality[6] [%] | 1.1 | 0.0 | 0.8 | | 0.357 | | |
| Mortality+culling[6] [%] | 1.5 | 0.0 | 0.8 | | 0.347 | | |
| **Grower (d10-d21)** | | | | | | | |
| Final BW [g] | 1174 | 1189 | 1194 | 4.3 | **0.066** | 0.027 | 0.453 |
| ADG [g/d] | 73.6[b] | 75.5[a] | 76.1[a] | 0.35 | **0.004** | **0.002** | 0.320 |
| ADFI [g/d] | 105 | 103 | 102 | 0.52 | 0.171 | **0.085** | 0.454 |
| F:G | 1.42[a] | 1.36[b] | 1.35[b] | 0.009 | <0.001 | <0.001 | 0.104 |
| Mortality[6] [%] | 0.0 | 1.1 | 0.4 | | 0.147 | | |
| Mortality+culling[6] [%] | 0.4 | 1.1 | 0.8 | | 0.558 | | |
| **Finisher (d21-d39)** | | | | | | | |
| Final BW, g | 3081 | 3100 | 3066 | 11.5 | 0.491 | 0.566 | 0.326 |
| ADG, g/d | 106 | 106 | 104 | 0.6 | 0.212 | 0.174 | 0.333 |
| ADFI, g/d | 177 | 179 | 178 | 1.0 | 0.775 | 0.731 | 0.609 |
| $FI_{HS}$[5], % | 26.2 | 27.9 | 27.9 | 0.43 | 0.134 | 0.129 | 0.368 |
| F:G | 1.69 | 1.71 | 1.72 | 0.008 | 0.269 | 0.108 | 0.907 |
| Mortality[6] [%] | 6.3 | 4.7 | 3.1 | | 0.399 | | |
| Mortality+culling[6] [%] | 7.6 | 6.0 | 3.1 | | 0.160 | | |
| **Total (d0-d39)** | | | | | | | |
| ADG, g/d | 77.9 | 78.4 | 77.6 | 0.30 | 0.495 | 0.569 | 0.329 |
| ADFI, g/d | 116 | 116 | 117 | 0.6 | 0.842 | 0.627 | 0.918 |
| F:G | 1.54 | 1.53 | 1.54 | 0.004 | 0.891 | 0.912 | 0.641 |
| F:G corrected[7] | 1.54 | 1.54 | 1.54 | 0.004 | 0.802 | 0.523 | 0.879 |
| Mortality[6] [%] | 7.4 | 5.9 | 4.2 | | 0.472 | | |
| Mortality+culling[6] [%] | 9.5 | 7.3 | 4.5 | | 0.246 | | |

(continued)

| Total (d0-d39) | | | | | | | |
|---|---|---|---|---|---|---|---|
| EPEF[8] | 476 | 488 | 491 | 6.4 | 0.561 | 0.362 | 0.761 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39

[2] Values with different superscripts within a row are significantly different at $p<0.05$

[3] T1: control diet, T2: control diet + 0.1% DMS, and T3: control diet + 0.2% DMS

[4] Standard Error of the Mean

[5] Indicates the percentage of total feed intake that was consumed during hours of heat stress (6 h per day) in period d-24 till d-39. Statistics was done including day as within subject factor (repeated measure)

[6] Data within treatment were not normally distributed, and hence treatments were compared using the non-parametric Kruskal-Wallis test

[7] F:G for total period corrected to final body weight of 3050 g, by assuming 1.76 points increase per 100 g body weight (based on Male Ross 308 Performance Objectives 2019)

[8] European Production Efficiency Factor, $EPEF = \frac{(100-MR) \times BW_{d-39}}{39 \times F:G} \times 100$

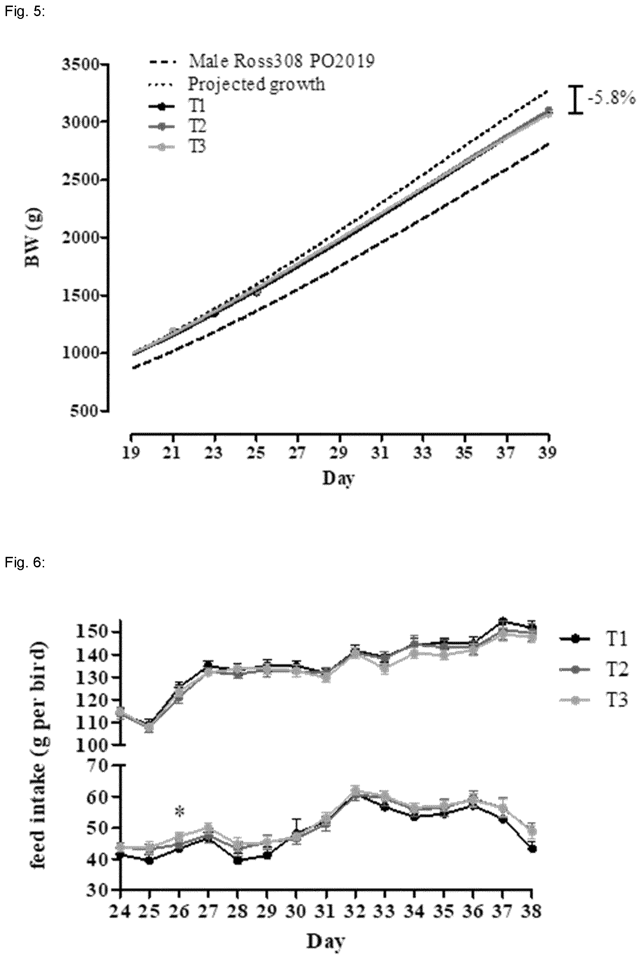

Fig.5 explains the effect of the chronic cyclic heat stress on birds' growth better. It shows the growth from d-19 to d-39, thus emphasizing growth in late grower and finisher phase. First, growth according to male Ross 308 Performance Objectives 2019 is given (full black line). Obviously, treatments T1-T3 (grayscale lines) show much higher growth, but as described above, no differences amongst treatments were noticed for body weight. Next a projected growth is established (broken black line) suggesting growth in thermo-neutral conditions. This is done by connecting the average body weight of all treatments at d-21 (end of grower phase, no heat stress yet established) with an estimated body weight at d-39 in case there would be no heat stress. This estimation is based on the following assumptions according to a meta-analysis of 43 published papers (83 challenges) including studies with a thermo-neutral and heat stress treatment:

-    the meta-analysis reveals that ADG is reduced by 2.1 g/d for every °C above thermo-neutral,
-    for chronic cyclic heat stress models, the difference between thermo-neutral and high temperature is halved, *in casu* this is then (34-22)/2 = 6 °C,
-    in current experiment, the chronic cyclic heat stress was implemented for 15 days,
-    altogether, it can be calculated that the reduction in body weight at d-39 equals 189 g.

The average body weight at d-39 observed for treatments T1-T3 was 3083 g, which means that the estimated body weight at d-39 in case there would be no heat stress would be 3272 g. In other words, the implementation of the chronic cyclic heat stress model reduced final body weight by 189 g or 5.8%.

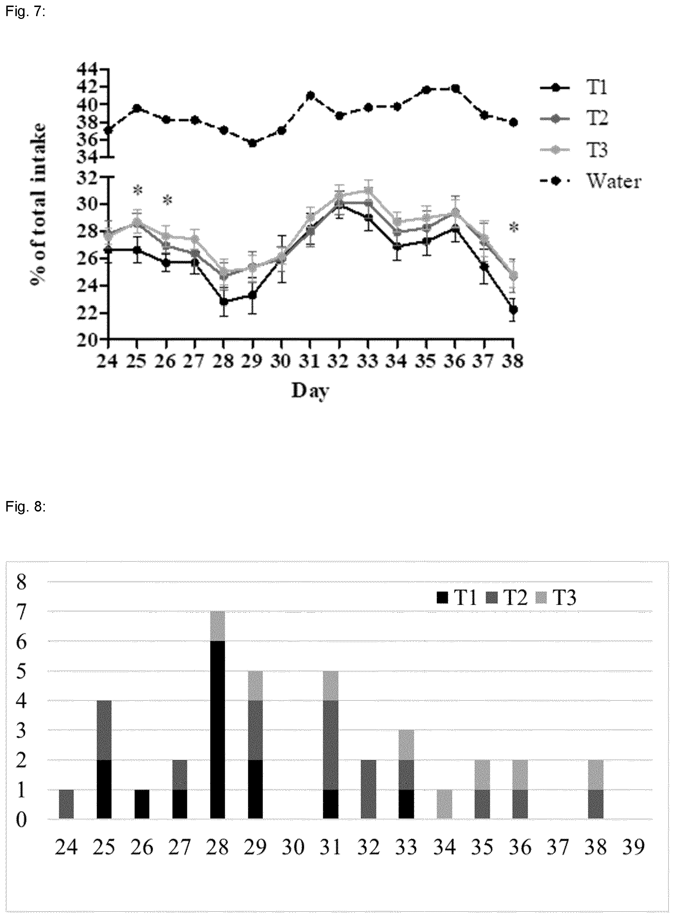

In Table 3 it was shown that parameters ADFI and $FI_{HS}$ in finisher period did not differ significantly among treatments. Fins indicates the percentage of total feed intake that was consumed during hours of heat stress (6 h per day) in period d-24 till d-39. Fig. 6 and 7 give more details on the consumption of feed and water on a daily basis in period d-24 till d-39. Fig. 6 shows the absolute feed intake per bird and per day during period of 6 h of heat stress and during rest of the day. The intake during the rest of the day shows a more or less steady increase over the 15 days of heat stress protocol, which corresponds to normal increase in feed intake when birds become heavier. The intake during period of 6 h of heat stress shows more inconsistent fluctuations, with an almost constant intake until d-31, after which it increased before dropping again on d-37 and d-38. It should be noted that this almost perfectly mirrors the actual temperature in the stable. As said above (see Fig. 2), initially the high temperature was set to 34°C, but by d-32 it was reduced to 32°C, and subsequently increased again on d-38 to 33°C. It is very obvious that the actual room temperature had an immediate effect on feed intake during this high temperature hours. Importantly to observe is that on most days the feed intake during period of 6 h of heat stress was higher in supplemented groups as compared to control, which resulted in a linear trend on d-26 only. It might indicate that supplemented birds suffered less from heat increment induced by feed consumption. This is even more clear when looking at Fig. 7. Relative feed intake during period of 6 h of heat stress fluctuated between 22 and 30%, and on several days a linear trend was observed by incremental levels of DMS in the diet. To understand this figure, the light schedule was 18L:6D, which means that the lights-on duration per day was 18 h of which 6 h with heat stress. Feed intake during heat stress might thus theoretically correspond to one third of total intake. Fig. 7 shows clearly that this is much lower (between 22 and 30%), thus indicating reduced feed intake by the heat stress protocol. In Table 3 it was already seen that FI$_{HS}$ for supplemented groups was numerically lower than control, thus suggesting higher heat tolerance. Not surprisingly, relative water intake during period of 6 h of heat stress (measured for all pens together) is much higher than one third, actually exceeding 35%, and in some days up to 42%. Thus, birds reduce feed intake whilst massively increase water consumption during heat stress episodes.

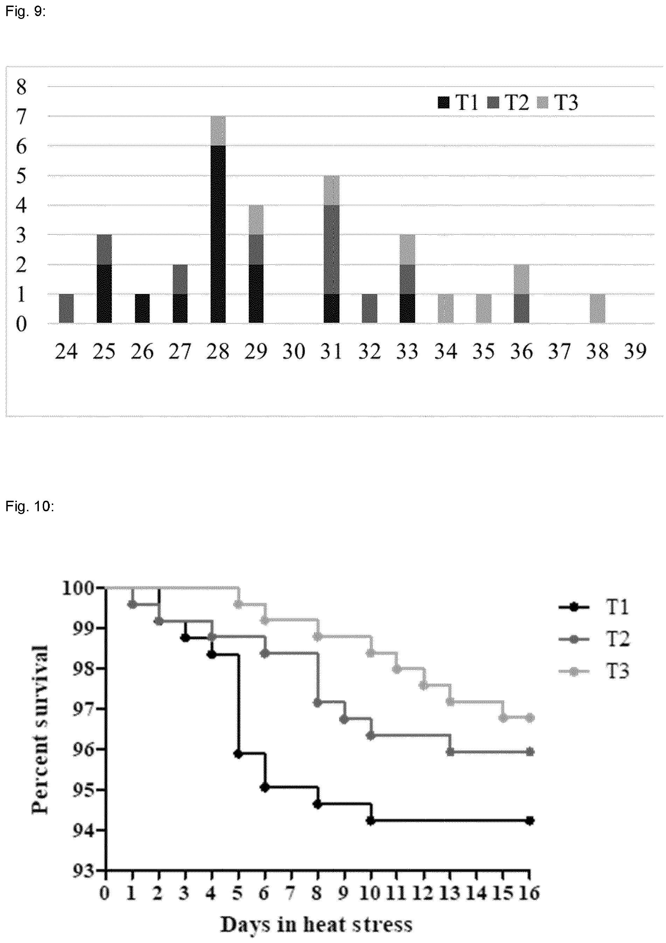

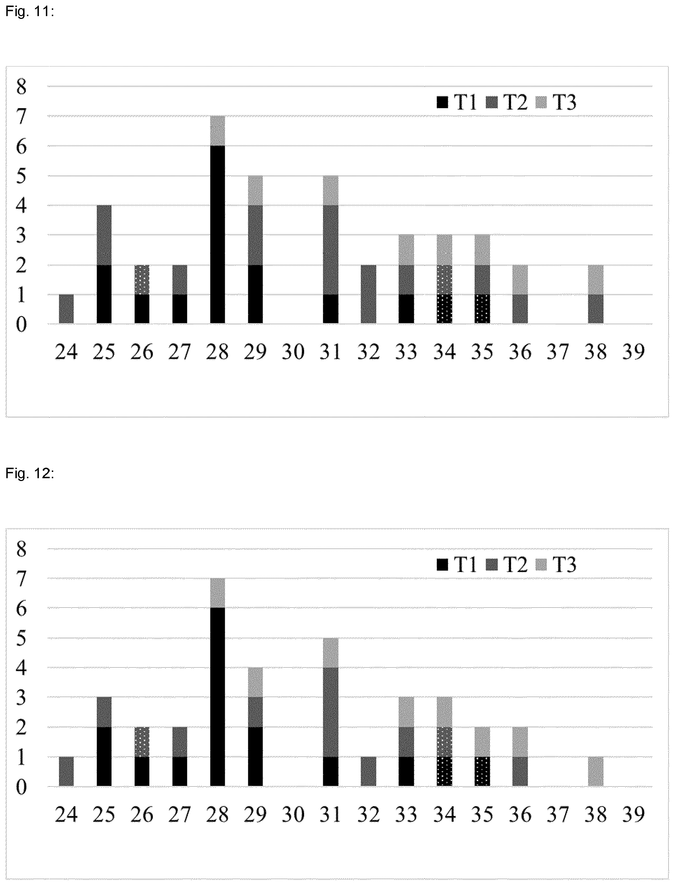

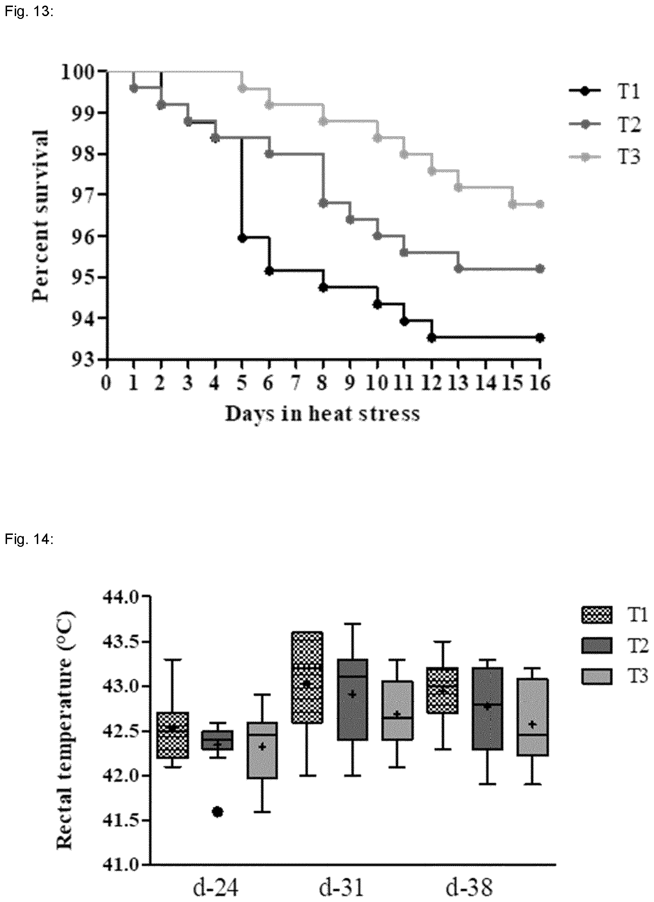

Above it was highlighted that supplementing DMS appeared to increase heat tolerance and thus reduce mortality. According to Table 3, mortality in finisher period accounted for 6.3, 4.7 and 3.1% in T1, T2 and T3, respectively. The following figures detail all cases of mortality and culling in the finisher period, either or not excluding pen 24 (Fig. 8-13). As an example, Fig. 9 is discussed. In this figure, number of cases of mortality were 14, 10 and 8 for T1, T2 and T3, respectively. Beside the fact that the number is lower in DMS supplemented groups, it is interesting to see that mortality cases in T1 mostly occur in the beginning of the heat stress period whereas in T3 dead birds were found mostly in the second half of this period. T2 has mortality cases across all days. This is further illustrated in Fig. 10 by plotting survival curves for the period with heat stress implemented. It lends to the speculation that supplementing DMS gives birds most benefits during the acute phase of a heat wave (first few days), whereas it might not add advantage in more chronically heat stressed birds. Analogously, Fig. 12 and 13 give mortality+culling. Number of cullings was limited by only in T1 and T2. Here, statistics on Fig. 13 says there was a trend for differences in survival curves. It can also be speculated whether this difference in timing of mortality might have affected calculations for ADFI and F:G in the finisher period. For instance, F:G increases when birds are older, thus having more dead birds at the end of finisher period would overestimate F:G, or in other words it could be suspected that F:G for finisher is underestimated for T1 and overestimated for T3. If this is the case, the true F:G would be more in favour for T3, and not numerically higher for T3 as compared to T1 as seen in Table 3. However, authors did not find a method to correct for this and recalculate F:G in one way or another.

Table 4 shows the effect of treatment on rectal temperature and panting on days 24, 31 and 38 of the study. Both parameters were measured during the last 2 hours of the 6-h heat stress episode on the respective days, and illustrate that birds suffered from high environmental temperatures. Regarding rectal temperature, numerically, supplementation with DMS resulted in lower rectal temperatures at all days, supporting the idea that heat tolerance was improved. Fig. 14 illustrates these effects. On d-38, it resulted in a linear effect (P<0.05). In total, rectal temperature was significantly reduced by 0.2% DMS in the diet as compared to control (P<0.05). To note, rectal temperature of birds at thermo-neutral fluctuates between 40.5 and 41.5°C. Panting occurs heavily during episodes of heat stress, and apparently increased when birds' age, although d-38 was not higher than d-31, likely because room temperature was 33 and 34°C for these days, respectively. At d-24, a significant treatment effect was found which indicates that panting frequency was reduced by 0.2% DMS as compared to control, and the effect at d-24 was linear (P<0.05). Figures for d-31 and d-38 were not significant, but for total again a numerical lower panting frequency was observed. Respiratory frequency of birds at thermo-neutral would be between 40-80, depending on age and metabolism. The fact that in particular at d-24 differences were more pronounced (and significant) for both rectal temperature and panting might indicate that DMS supplemented birds were better prepared in the previous feeding period, hereby referring to improved feed conversion ratio in the grower period, to cope with the upcoming heat stress. It looks also that towards the end of the heat stress period, enhanced heat tolerance was less visible. This corresponds to findings about mortality as discussed above.

**Table 4. Dietary effect on rectal temperature and panting in male broilers subjected to chronic cyclic heat stress in the finisher phase[123] (n=10-12, i.e. 10-12 pen replicates per treatment).**

| Item | T1 | T2 | T3 | SEM[4] | P | | |
|---|---|---|---|---|---|---|---|
| | | | | | Model | Linear | Quadratic |
| **Rectal temperature** [°C] | | | | | | | |
| d-24 | 42.5 | 42.3 | 42.3 | 0.06 | 0.304 | 0.154 | 0.540 |
| d-31 | 43.0 | 42.9 | 42.6 | 0.09 | 0.252 | 0.106 | 0.761 |
| d-38 | 42.9 | 42.7 | 42.6 | 0.07 | 0.118 | **0.040** | 0.990 |
| Total[5] | 42.8[a] | 42.6[ab] | 42.5[b] | 0.03 | **0.013** | | |
| **Panting, #/min** | | | | | | | |
| d-24 | 134[a] | 119[ab] | 112[b] | 4.1 | **0.035** | **0.012** | 0.508 |
| d-31 | 154 | 158 | 143 | 4.4 | 0.347 | 0.315 | 0.292 |
| d-38 | 153 | 147 | 152 | 2.9 | 0.638 | 0.894 | 0.350 |

(continued)

| Panting, #/min | | | | | | | |
|---|---|---|---|---|---|---|---|
| Total[5] | 148 | 141 | 137 | 2.8 | 0.200 | | |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39
[2] Values with different superscripts within a row are significantly different at $p<0.05$
[3] T1: control diet, T2: control diet + 0.1% DMS, and T3: control diet + 0.2% DMS
[4] Standard Error of the Mean
[5] For all days, with statistics done including day as within subject factor (repeated measure)

2.4 Carcass yield and breast meat characteristics

No significant effects on carcass yield and portions were found (Table 5). Effect on breast meat characteristics are shown in Table 6. pH of breast at slaughter and 24 h post-mortem was not changed by the diet. Regarding colour, consistent effects on L* and b* might be conceived, though only significance was seen for b* 24 h post-mortem.

Table 5. Dietary effect on carcass yield and portions in male broilers subjected to chronic cyclic heat stress in the finisher phase[123] after overnight fasting on d-40 (n=10-12, i.e. 10-12 pen replicates per treatment).

| Item | T1 | T2 | T3 | SEM[4] | P | | |
|---|---|---|---|---|---|---|---|
| | | | | | Model | Linear | Quadratic |
| Body weight, kg | 2902 | 3007 | 2983 | 24.3 | 0.191 | 0.170 | 0.215 |
| Abdominal fat, % | 1.1 | 1.1 | 1.1 | 0.05 | 0.996 | 0.878 | 0.838 |
| Carcass yield, % | 75.9 | 74.9 | 75.9 | 0.26 | 0.196 | 0.983 | 0.073 |
| Breast percentage [%] | 24.2 | 23.4 | 24.1 | 0.19 | 0.182 | 0.784 | 0.069 |
| Thigh percentage [%] | 11.2 | 11.1 | 11.2 | 11.2 | 0.11 | 0,986 | 0.966 |
| Drumstick [%] | 9.9 | 10.0 | 10.2 | 0.07 | 0.07 | 0.129 | 0.961 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39
[2] Values with different superscripts within a row are significantly different at $p<0.05$
[3] T1: control diet, T2: control diet + 0.1% DMS, and T3: control diet + 0.2% DMS
[4] Standard Error of the Mean

Table 6. Dietary effect on breast characteristics in male broilers subjected to chronic cyclic heat stress in the finisher phase[123] after overnight fasting on d-40 (n=10-12, i.e. 10-12 pen replicates per treatment).

| Item | | T1 | T2 | T3 | SEM[4] | P | | |
|---|---|---|---|---|---|---|---|---|
| | | | | | | Model | Linear | Quadratic |
| Body weight [kg] | | 2902 | 3007 | 2983 | 24.3 | 0.191 | 0.170 | 0.215 |
| pH 0h | | 6.8 | 6.8 | 6.8 | 0.03 | 0.853 | 0.796 | 0.615 |
| pH 24h | | 6.0 | 6.0 | 6.0 | 0.02 | 0.385 | 0.517 | 0.229 |
| Colour | L* 0h | 57.9 | 56.7 | 56.9 | 0.51 | 0.613 | 0.425 | 0.550 |
| | a* 0h | 3.2 | 3.6 | 3.5 | 0.19 | 0.759 | 0.539 | 0.672 |
| | b* 0h | 15.9 | 15.4 | 15.5 | 0.26 | 0.734 | 0.541 | 0.616 |
| Colour | L* 24h | 57.8 | 56.7 | 56.2 | 0.59 | 0.549 | 0.283 | 0.849 |

(continued)

| Item | | T1 | T2 | T3 | SEM[4] | P | | |
|---|---|---|---|---|---|---|---|---|
| | | | | | | Model | Linear | Quadratic |
| | a* 24h | 5.0 | 5.0 | 5.3 | 0.16 | 0.751 | 0.556 | 0.650 |
| | b* 24h | 18.1[a] | 16.7[b] | 17.3[ab] | 0.22 | 0.026 | 0.116 | 0.022 |
| Drip loss | 0-24h [%] | 2.3 | 2.0 | 1.8 | 0.19 | 0.615 | 0.329 | 0.921 |
| | 0-72h [%] | 3.1 | 2.7 | 2.7 | 0.21 | 0.673 | 0.407 | 0.728 |
| Press loss [%] | | 20.3 | 20.4 | 20.4 | 0.31 | 0.987 | 0.875 | 0.994 |
| Wooden breast 0h[5] | | 21/1/0 | 19/3/0 | 22/2/0 | | 0.565 | | |
| Wooden breast 24h[5] | | 14/8/0 | 15/7/0 | 12/12/0 | | 0.420 | | |
| White striations 0h[6] | | 19/3/0 | 21/1/0 | 20/4/0 | | 0.420 | | |
| White striations 24h[6] | | 21/1/0 | 21/1/0 | 18/6/0 | | 0.044 | | |
| TBARS [μg MDA/g] | | 0.157 | 0.125 | 0.122 | 0.0083 | 0.155 | 0.078 | 0.406 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39
[2] Values with different superscripts within a row are significantly different at $p<0.05$
[3] T1: control diet, T2: control diet + 0.1% DMS, and T3: control diet + 0.2% DMS
[4] Standard Error of the Mean
[5] Wooden breast: normal, 0; mild, 1 and severe, 2. Count per level are given in order of severity and statistical evaluation was done by the Chi-square test (n=22-24).
[6] White striations: normal, 0; mild, 1, and severe, 2. Count per level are given in order of severity and statistical evaluation was done by the Chi-square test (n=22-24).

2.5 Physiological measurements in birds sampled at d-23, d-25 and d-39

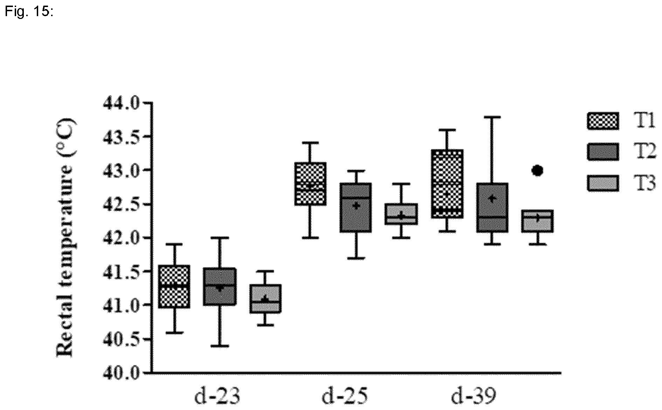

At d-23, d-25 and d39, one bird per pen (with weight close to average weight of the pen) was sampled. On d-25 and d-39, sampling started after >3h of heat stress that day. Thus, in total 108 birds were sampled, and various endpoints were determined in several tissues. Table 7 reports the body weight and rectal temperature of birds sampled. To recall, finisher period started on d-21, while the heat stress protocol was implemented from d-24 onwards. It means that d-23 was the last day prior to heat stress and d-25 the second day whereby birds were subjected to high temperatures. Body weights on d-23 and d-25 (Table 7) were naturally higher than those reported on d-21 (Table 3), but not affected by treatment. Body weights at d-39 for sampled birds are very close to body weights for d-39 shown in Table 3, which confirms that birds with weight close to average weight of the pen were selected. Again, this was not affected by supplementation with DMS. Rectal temperature of sampled birds on d-23 represents values for thermo-neutral conditions, and thus ranged between 41.1 and 41.3°C without treatment differences (Fig. 15). However, on d-25 rectal temperatures were largely elevated and obviously affected by diet (P<0.05). Rectal temperature linearly decreased with higher DMS in the diet, both supplemented groups being different from control, and for T3 this was 0.5°C lower than control. This rectal temperature is slightly higher than those on d-24 as shown in Table 4, at least for T1 and T2, suggesting increased disturbance of body temperature homeostasis in these groups. Rectal temperature on d-39 again drops with higher DMS in the diet but did not reach significance. Nonetheless, observations are in line with findings on d-38 (Table 4).

**Table 7. Dietary effect on body weight and rectal temperature in male broilers subjected to chronic cyclic heat stress in the finisher phase[123] on d-23, d-25 and d-39 sampled between 4 and 6-7 h of heat stress on the respective day (n=10-12, i.e. 10-12 pen replicates per treatment).**

| Item | T1 | T2 | T3 | SEM[4] | p | | |
|---|---|---|---|---|---|---|---|
| | | | | | Model | Linear | Quadratic |
| **Body weight, g** | | | | | | | |
| d-23 | 1341 | 1361 | 1352 | 5.9 | 0.421 | 0.474 | 0.271 |
| d-25 | 1535 | 1544 | 1536 | 6.3 | 0.827 | 0.957 | 0.542 |
| d-39 | 3071 | 3097 | 3068 | 15.0 | 0.638 | 0.846 | 0.500 |
| **Rectal temperature, °C** | | | | | | | |
| d-23 | 41.3 | 41.3 | 41.1 | 0.06 | 0.358 | 0.183 | 0.604 |
| d-25 | 42.8[a] | 42.5[b] | 42.3[b] | 0.07 | **0.005** | **0.006** | 0.544 |
| d-39 | 42.7 | 42.6 | 42.3 | 0.09 | 0.263 | 0.127 | 0.571 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39
[2] Values with different superscripts within a row are significantly different at $p<0.05$
[3] T1: control diet, T2: control diet + 0.1% DMS, and T3: control diet + 0.2% DMS
[4] Standard Error of the Mean

**Table 8. Dietary effect on parameters in blood in male broilers subjected to chronic cyclic heat stress in the finisher phase[123] on d-23, d-25 and d-39 sampled between 4 and 6 h of heat stress on the respective day (n=10-12, i.e. 10-12 pen replicates per treatment).**

| Item | Matrix | T1 | T2 | T3 | SEM[4] | p | | |
|---|---|---|---|---|---|---|---|---|
| | | | | | | Model | Linear | Quadratic |
| **Heat shock protein 70 [ng/mL]** | | | | | | | | |
| d-23 | | 1.69 | 1.69 | 1.00 | 0.164 | 0.139 | **0.084** | 0.323 |
| d-25 | Plasma | 1.09 | 1.07 | 1.08 | 0.077 | 0.995 | 0.983 | 0.926 |
| d-39 | | 3.63 | 2.77 | 4.23 | 0.438 | 0.406 | 0.580 | 0.223 |
| **T4 [µg/dL]** | | | | | | | | |
| d-23[5] | | | | | | | | |
| d-25 | Serum | 0.50 | 0.53 | 0.50 | 0.019 | 0.771 | 0.984 | 0.475 |
| d-39 | | 0.54 | 0.60 | 0.63 | 0.022 | **0.227** | **0.091** | 0.793 |
| **T3 [µg/L]** | | | | | | | | |
| d-23 | | 2.50[a] | 2.43[a] | 2.06[b] | 0.065 | **0.009** | **0.005** | 0.215 |
| d-25[6] | Serum | 1.50 | 1.74 | 1.72 | 0.064 | 0.199 | 0.139 | 0.306 |
| d-39 | | 0.97 | 1.10 | 1.12 | 0.032 | **0.094** | **0.059** | 0.618 |
| **T3/T4** | | | | | | | | |
| d-23[5] | | | | | | | | |
| d-25[6] | Serum | 0.304 | 0.342 | 0.351 | 0.0160 | 0.421 | 0.219 | 0.660 |
| d-39 | | 0.180 | 0.193 | 0.180 | 0.0089 | 0.790 | 0.793 | 0.716 |

(continued)

| Nitric oxide [$\mu$mol/L] | | | | | | | | |
|---|---|---|---|---|---|---|---|---|
| d-23 | Serum | 5.26 | 6.02 | 6.06 | 0.394 | 0.574 | 0.421 | 0.528 |
| d-25 | | 3.30 | 4.31 | 4.48 | 0.330 | 0.308 | 0.149 | 0.561 |
| d-39 | | 3.24 | 3.84 | 3.14 | 0.358 | 0.713 | 0.915 | 0.420 |
| Malondialdehyde [mmol/mL] | | | | | | | | |
| d-23 | Plasma | 14.7 | 14.4 | 14.2 | 0.26 | 0.785 | 0.492 | 0.951 |
| d-25 | | 14.8 | 14.3 | 13.2 | 0.28 | **0.057** | **0.020** | 0.592 |
| d-39 | | 17.1 | 15.7 | 14.9 | 0.42 | **0.076** | **0.025** | 0.698 |
| Glutathione peroxidase [U/mL] | | | | | | | | |
| d-23 | Plasma | 0.85 | 0.82 | 0.92 | 0.051 | 0.687 | 0.547 | 0.537 |
| d-25 | | 0.81 | 0.84 | 0.86 | 0.032 | 0.815 | 0.527 | 0.942 |
| d-39 | | 1.00 | 0.97 | 1.09 | 0.033 | 0.291 | 0.239 | 0.311 |
| Super oxide dismutase [% inhibition] | | | | | | | | |
| d-23 | Plasma | 79.9 | 84.1 | 80.4 | 2.24 | 0.709 | 0.926 | 0.413 |
| d-25 | | 84.8 | 81.3 | 84.1 | 1.86 | 0.721 | 0.883 | 0.430 |
| d-39 | | 88.0 | 79.5 | 87.1 | 2.18 | 0.236 | 0.847 | **0.093** |
| Glutathione (GSH) $\mu$mol/mL | | | | | | | | |
| d-23 | Erythrocytes | 0.91 | 1.07 | 1.25 | 0.066 | 0.103 | **0.035** | 0.930 |
| d-25[6] | | 0.59 | 0.59 | 0.70 | 0.026 | **0.088** | **0.052** | 0.303 |
| d-39 | | 0.50[b] | 0.64[ab] | 0.67[a] | 0.031 | **0.037** | **0.018** | 0.311 |
| Oxidized glutathione (GSSG) [$\mu$mol/mL] | | | | | | | | |
| d-23 | Erythrocytes | 0.016 | 0.017 | 0.023 | 0.0020 | 0.322 | 0.167 | 0.560 |
| d-25 | | 0.009 | 0.010 | 0.009 | 0.0005 | 0.921 | 0.886 | 0.707 |
| d-39 | | 0.009 | 0.011 | 0.010 | 0.0006 | 0.220 | 0.564 | 0.12 |
| GSSG/GSH | | | | | | | | |
| d-23 | Erythrocytes | 0.018 | 0.015 | 0.018 | 0.0011 | 0.600 | 0.887 | 0.321 |
| d-25 | | 0.016[ab] | 0.017[a] | 0.013[b] | 0.0006 | **0.027** | **0.054** | **0.049** |
| d-39 | | 0.017 | 0.017 | 0.015 | 0.0007 | 0.454 | 0.446 | 0.446 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39

[2] Values with different superscripts within a row are significantly different at $p < 0.05$

[3] T1: control diet, T2: control diet + 0.1% DMS, and T3: control diet + 0.2% DMS

[4] Standard Error of the Mean

[5] Many values were below detection limit of 0.42 $\mu$g/dL, hence statistical analysis could not be done

[6] Variable was significantly negatively influenced by time of sampling

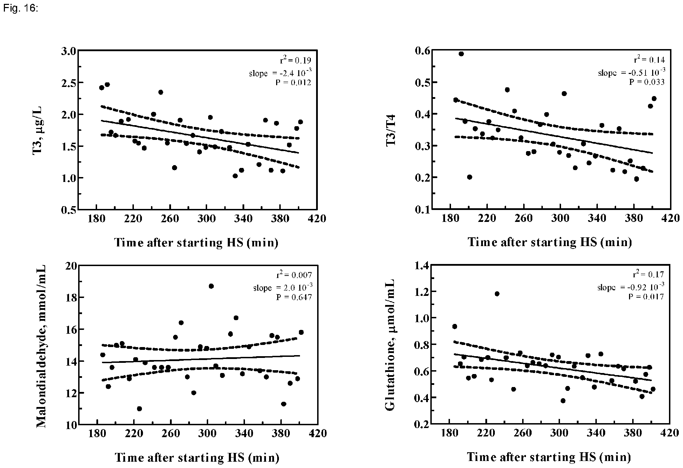

Parameters in blood are given in Table 8. For some cases, the time after starting heat stress that day affected linearly the response for the outcome (Fig. 16). In particular, T3, T3/T4, and glutathione were all decreasing when time advanced irrespective of treatment (all $P < 0.05$). To clarify, malondialdehyde was not affected by time after starting heat stress but is given in Fig. 16 as example. The graphs also show that only a small fraction of the variance is explained by time after starting heat stress, $r^2$ is ranging between 0.14 and 0.19. Nonetheless, the decrease in T3, and coherently in T3/T4 can be regarded as an adaptation to reduce body metabolism and hitherto metabolic heat production. This view might be supported by the fact that T3 values sharply dropped when comparing d-23, d-25 and d-39 (Table 8). It means that when heat stress was prolonged for 2 weeks, a continuous decrease in T3 and its activation from T4 can be observed. As no thermo-neutral control was included in the study, the effect of age cannot be ruled out. However, the reduction is strong and moreover T4 does not seem to be affected by day. Also, glutathione declines with time after starting heat stress, which might be caused by higher needs in other tissues such as small intestine and respiratory system to foster antioxidant function and redox homeostasis, or by reduced synthesis primarily taken place in liver, as heat stress continues. Interestingly, also glutathione in erythrocytes seems to be heavily affected by implementing the heat stress protocol as values on d-23 are between 0.91-1.25 $\mu$mol/L whereas from d-25 onwards these do not exceed 0.70 $\mu$mol/L (Table 8). Besides redox imbalance is not anticipated since GSSG/GSH remain fairly stable across sampling days, thus merely the synthesis of glutathione is impacted.

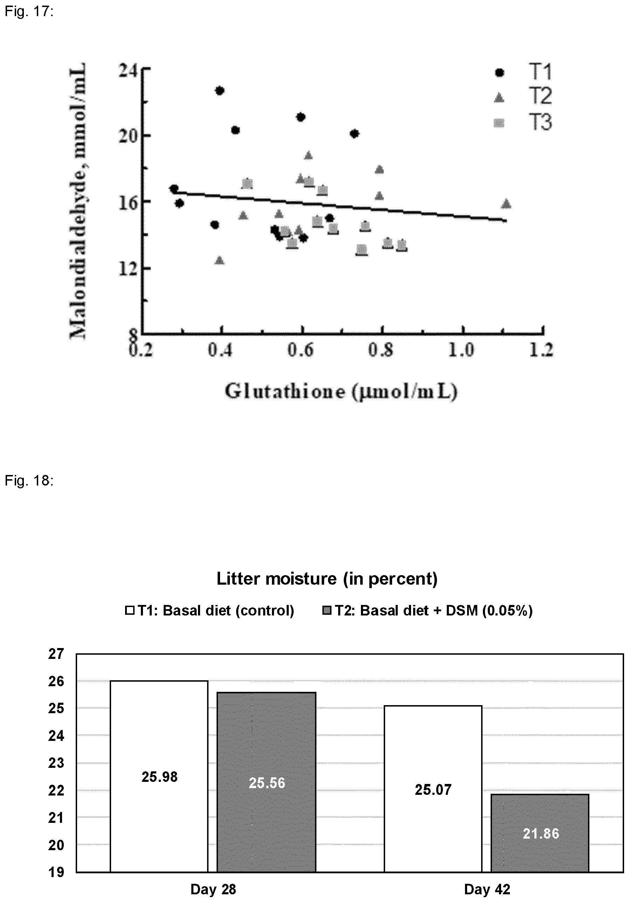

Heat shock protein 70 and heat shock protein 90 were determined in plasma employing the competitive inhibition enzyme immunoassay technique (HSP70, *Chicken Heat Shock Protein 70, Hsp-70 ELISA Kit; Cusabio Code CSB-E11196Ch)* and the quantitative sandwich enzyme immunoassay technique (HSP90, *Chicken Heat Shock Protein 90, HSP-90 ELISA Kit; Cusabio Code* CSB-E12873C), respectively. Regarding HSP-90 very low absorbances were found and in most cases fell below the lowest standard dilution. Values were then typically less than 0.39 ng/mL, the minimum detectable dose as mentioned in the user manual. For that reason, no data for HSP-90 could be reported here. Opposite to that, HSP-70 worked properly and values were all above sensitivity of 0.125 ng/mL. No treatments effects on HSP-70 could be perceived on either sampling day, apart from a trend for linear reduction on d-23. It also appears that plasma HSP-70 showed slight decrease upon acute heat stress (d-25), whereas after two weeks of heat stress (d-39) levels rose substantially. It must be noted that large variation across samples was found on d-39. Thyroxine (T4) could not be detected on d-23, while higher values were found on d-25 and d-39. A trend for linear increase on d-39 by higher DMS dose was observed. Triiodothyronine (T3), the derivative of T4 and most active form, was reduced on d-23 by feeding 0.2% DMS as compared to control ($P<0.05$). On d-39, a trend suggests that T3 is elevated by DMS, which is actually a result of higher T4 levels as the ratio T3/T4 was not affected. It means that conversion efficiency was not altered by diet. Irrespective of treatment is the sharp reduction in circulating T3 levels as birds age and thus subjected for long period to heat stress. As indicated above, this can be regarded as an adaptation to reduce body metabolism and hitherto metabolic heat production, and apparently birds fed DMS seems to be less affected. Nitric oxide was not different across treatments at any sampling day. Further, interesting changes in malondialdehyde, a marker of lipid peroxidation, were found. First, on both d-25 and d-39 a linear decrease in this metabolite was found when feeding DMS (both $P<0.05$), which suggests lower oxidative stress and/or higher antioxidant capacity. Remarkable is also the large increase in malondialdehyde from d25- to d-39 in T1, something that did not occur in DMS-fed chickens. The antioxidant enzymes glutathione peroxidase and superoxide dismutase were not changed by diet, and thus do not explain the effect on malondialdehyde. In contrast, glutathione (GSH) was on all days increased by adding DMS to the diet, i.e. a linear increase on d-23 ($P<0.05$), a trend for linear increase on d25, and a linear increase on d-39 ($P>0.05$) showing that 0.2% DMS resulted in higher GSH content in erythrocytes as compared to control. Said above, glutathione in erythrocytes seems to be heavily affected by implementing the heat stress protocol as values on d-23 are between 0.91-1.25 $\mu$mol/L whereas from d-25 onwards these do not exceed 0.70 $\mu$mol/L. Besides redox imbalance is not anticipated since GSSG/GSH remain fairly stable across sampling days, thus merely the synthesis of glutathione is impacted. The higher GSH in DMS-fed chickens thus promotes antioxidant function and redox homeostasis. However, no relation could be established between plasma malondialdehyde and erythrocyte glutathione (Fig. 17).

In liver, only glutathione peroxidase activity was different across treatments (Table 9). A linear decrease was demonstrated ($P<0.05$), though differences small. Further noteworthy is the decline in GSH levels in aging birds, which has been repeatedly shown in this heat stress model. It implies that GSH synthesis and salvation - liver is main site of synthesis - becomes limiting, and/or export to tissues that require GSH is increased. Both hypotheses may hold truth. Firstly, in Table 9 it can be seen that the ratio GSSG to GSH from d-25 to d-39 tremendously increases. This suggest that salvation, i.e. the reduction of GSSG to GSH via glutathione reductase is hampered, leading to redox disturbance. Secondly, Table 10 indicates that GSH levels in breast muscle increases with age, from 1.03-1.10 to 1.39-1.56 $\mu$mol/g (d-23 to d-39). Knowing that breast muscle mass enormously increases in this growth period, it must mean that large amounts of GSH are needed to supply muscle. The high demand by breast muscle likely affects levels in liver negatively. Interestingly in Table 10, GSH was elevated linearly by DMS on d-25 ($P<0.05$) and a trend suggests the same for d-39. Surprisingly, on d-23, a decrease in malondialdehyde was found ($P<0.05$) and not on the other days.

**Table 9. Dietary effect on parameters in liver in male broilers subjected to chronic cyclic heat stress in the finisher phase[123] on d-23, d-25 and d-39 sampled between 4 and 6 h of heat stress on the respective day (n=10-12, i.e. 10-12 pen replicates per treatment).**

| Item | T1 | T2 | T3 | SEM[4] | p | | |
|---|---|---|---|---|---|---|---|
| | | | | | Model | Linear | Quadratic |
| **Malondialdehyde [mmol/g]** | | | | | | | |
| d-23 | 46.0 | 43.9 | 44.6 | 0.70 | 0.477 | 0.477 | 0.343 |
| d-25 | 51.0 | 48.6 | 49.1 | 0.70 | 0.323 | 0.245 | 0.323 |
| d-39 | 49.3 | 46.4 | 48.0 | 0.97 | 0.355 | 0.916 | 0.440 |
| **Glutathione peroxidase [U/g]** | | | | | | | |
| d-23 | 17.9 | 17.0 | 16.9 | 0.20 | **0.091** | **0.043** | 0.395 |
| d-25 | 18.2 | 17.8 | 17.9 | 0.22 | 0.691 | 0.569 | 0.514 |
| d-39 | 18.2 | 17.5 | 18.6 | 0.29 | 0.303 | 0.535 | 0.163 |
| **Super oxide dismutase [U/g]** | | | | | | | |
| d-23 | 307 | 332 | 317 | 6.7 | 0.303 | 0.545 | 0.156 |
| d-25 | 274 | 292 | 289 | 6.7 | 0.507 | 0.342 | 0.489 |
| d-39 | 358 | 356 | 346 | 6.5 | 0.637 | 0.686 | 0.636 |
| **Glutathione (GSH) [μmol/g]** | | | | | | | |
| d-23 | 5.07 | 4.69 | 5.04 | 0.107 | 0.279 | 0.909 | 0.114 |
| d-25 | 4.06 | 4.26 | 4.14 | 0.105 | 0.676 | 0.697 | 0.578 |
| d-39 | 3.82 | 3.74 | 3.94 | 0.091 | 0.676 | 0.574 | 0.497 |
| **Oxidized glutathione (GSSG) [μmol/g]** | | | | | | | |
| d-23 | 0.081 | 0.077 | 0.076 | 0.0014 | 0.360 | 0.198 | 0.520 |
| d-25 | 0.076 | 0.070 | 0.069 | 0.0020 | 0.303 | 0.144 | 0.575 |
| d-39 | 0.099 | 0.093 | 0.097 | 0.0025 | 0.688 | 0.789 | 0.411 |
| **GSSG/GSH** | | | | | | | |
| d-23 | 0.016 | 0.016 | 0.015 | 0.0004 | 0.473 | 0.899 | 0.227 |
| d-25 | 0.018 | 0.017 | 0.017 | 0.0006 | 0.559 | 0.494 | 0.398 |
| d-39 | 0.026 | 0.027 | 0.025 | 0.0011 | 0.800 | 0.746 | 0.563 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39

[2] Values with different superscripts within a row are significantly different at $p < 0.05$

[3] T1: control diet, T2: control diet + 0.1% DMS, and T3: control diet + 0.2% DMS

[4] Standard Error of the Mean

**Table 10. Dietary effect on parameters in breast muscle in male broilers subjected to chronic cyclic heat stress in the finisher phase[123] on d-23, d-25 and d-39 sampled between 4 and 6 h of heat stress on the respective day (n=10-12, i.e. 10-12 pen replicates per treatment). Oxidized gluatathione (GSSG) was below detection limit (0.05 $\mu$mol/g) in samples of breast muscle and thus not reported here.**

| Item | T1 | T2 | T3 | SEM[4] | p | | |
|---|---|---|---|---|---|---|---|
| | | | | | Model | Linear | Quadratic |
| **Malondialdehyde [mmol/g]** | | | | | | | |
| d-23 | 5.72[a] | 4.72[b] | 4.50[b] | 0.185 | **0.011** | **0.005** | 0.280 |
| d-25 | 4.01 | 4.60 | 4.50 | 0.138 | 0.180 | 0.151 | 0.222 |
| d-39 | 5.42 | 5.23 | 5.57 | 0.246 | 0.881 | 0.808 | 0.662 |
| **Glutathione peroxidase [U/g]** | | | | | | | |
| d-23 | 0.40 | 0.39 | 0.38 | 0.008 | 0.763 | 0.483 | 0.839 |
| d-25 | 0.42 | 0.42 | 0.44 | 0.010 | 0.471 | 0.325 | 0.481 |
| d-39 | 0.45 | 0.46 | 0.50 | 0.022 | 0.274 | 0.133 | 0.605 |
| **Super oxide dismutase [U/g]** | | | | | | | |
| d-23 | 30.3 | 29.7 | 32.6 | 1.32 | 0.641 | 0.485 | 0.530 |
| d-25 | 28.6 | 27.3 | 28.1 | 1.14 | 0.893 | 0.862 | 0.658 |
| d-39 | 29.1 | 27.4 | 26.2 | 0.87 | 0.416 | 0.190 | 0.886 |
| **Glutathione (GSH) [$\mu$mol/g]** | | | | | | | |
| d-23 | 1.03 | 1.00 | 1.10 | 0.024 | 0.183 | 0.189 | 0.181 |
| d-25 | 1.12[b] | 1.30[ab] | 1.39[a] | 0.040 | **0.020** | **0.006** | 0.610 |
| d-39 | 1.39 | 1.46 | 1.56 | 0.039 | 0.175 | **0.066** | 0.835 |

[1] Broilers were fed a wheat-soybean starter diet from d-0 to d-10, a grower diet from d-10 to d-21 and a finisher diet from d-21 to d-39. The chronic cyclic heat stress model was implemented from d-24 till d-39
[2] Values with different superscripts within a row are significantly different at p<0.05
[3] T1: control diet, T2: control diet + 0.1% DMS, and T3: control diet + 0.2% DMS
[4] Standard Error of the Mean

**2.6 Litter moisture**

In addition, it was also studied whether the administration of dimethyl sulfone and/or of dimethyl sulfone comprising composition also has an impact on the moisture of the litter in the pens.
For this purpose, two treatments (T1: control diet, and T2: control diet + 0.05 wt.-% DMS) were done with 13 replicate pens each and 10 birds per pen. Collection of litter was done at days 28 and 42 to determine litter moisture. The sampling of the litter was done in five different points in each pen (each corner and the centre of each pen) and then homogenized to compose the representative sample of the pen. The moisture content in the litter was determined by weighing samples before and placing it in an oven at 103 °C for 24 hours. Calculating was done according to the formula:

$$litter\ moisture\ content\ [\%] = \frac{wet\ litter\ weight - dry\ litter\ weight}{wet\ litter\ weight} \times 100$$

The experiment showed a clear reduction in the moisture content in the litter at d-42, i.e. after the birds of the first experimental run were slaughtered and the pens were filled with new birds.

**3. Conclusions**

Supplementing DMS did not affect the performance in the starter phase. In contrast, in grower period, final body weight and ADG showed linear increases with higher inclusion of DMS in the diet, it was 2.6 and 3.4% higher with 0.1 and 0.2% DMS, respectively, as compared to control. This was associated by a remarkable improvement of feed conversion ratio. F:G was reduced by 6 and 7 points for 0.1 and 0.2% DMS, respectively, as compared to control. In finisher period, none of the performance indicators was affected by treatment. Nonetheless, a numerical reduction of mortality by supplementing DMS could clearly be observed, mortality accounted for 6.3, 4.7 and 3.1% in control, and 0.1 and 0.2% DMS, respectively. Besides the fact that number of dead cases was lower in DMS supplemented groups, it was interesting to see that mortality cases in control mostly occurred in the beginning of the HS period whereas in 0.2% DMS group dead birds were found mostly in the second half of this period. Treatment 0.1% DMS showed mortality cases across all days. This is further evidenced by reduced panting frequency and rectal temperature in birds fed DMS during heat stress. Moreover, the higher tolerance towards heat stress might be associated with following observations: 1/ a trend for linear increase of serum T3 on d-39 by higher DMS dose, likely illustration better metabolic adaptation to HS, 2/ on both d-25 and d-39 a linear decrease in plasma malondialdehyde, which suggests lower oxidative stress and/or higher antioxidant capacity, 3/ increased erythrocyte glutathione (GSH) on all days by adding DMS to the diet, promoting antioxidant function and redox homeostasis, and 4/ linearly elevated GSH in breast muscle by DMS on d-25 and a trend suggesting the same for d-39.

Supplementing DMS thus improved performance in grower period, with likely carry-over effects in the finisher phase that supported tolerance to heat, essentially reducing mortality when HS was applied. Various physiological observations support this finding.

## Claims

1. Dimethyl sulfone for use in the treatment of heat stress in poultry by oral administration, wherein the dimethyl sulfone is administered to chronically heat stressed poultry.

2. The dimethyl sulfone for use according to claim 1, wherein the poultry is exposed to a temperature of more than 27 °C, preferably to a temperature of at least 30°C.

3. The dimethyl sulfone for use according to claim 1 or 2, wherein the poultry is exposed to a temperature of more than 27 °C, preferably to a temperature of at least 30°C, for at least 5 hours a day.

4. The dimethyl sulfone for use according to any of claims 1 to 3, wherein the poultry is exposed to a relative air humidity of at least 40% on average.

5. The dimethyl sulfone for use according to any of claims 1 to 4, wherein the dimethyl sulfone is administered to poultry in the starter, grower and/or finisher phase.

6. A composition comprising dimethyl sulfone for use in the treatment of heat stress in poultry by oral administration, wherein the composition comprises at least 0.05 wt.-% of dimethyl sulfone, based on the total weight of the composition, and is administered to chronically heat stressed poultry.

7. The composition for use according to claim 6, wherein the composition comprises dimethyl sulfone in a concentration from 0.05 to 0.3 wt.-%, based on the total weight of the liquid and/or solid matrix of the composition.

8. The composition for use according to claim 6 or 7, wherein the composition is administered to poultry being exposed to a temperature of more than 27 °C, preferably to a temperature of at least 30°C.

9. The composition for use according to any of claims 6 to 8, wherein the composition is administered to poultry being exposed to a temperature of more than 27 °C, preferably to a temperature of at least 30°C, for at least 5 hours a day.

10. The composition for use according to any of claims 6 to 9, wherein the composition is administered to poultry being exposed to a relative air humidity of at least 40% on average.

11. The composition for use according to any of claims 6 to 10, wherein the composition is administered to poultry in the starter, grower and/or finisher phase.

12. The composition for use according to any of claims 6 to 11, wherein the composition is administered to poultry starting from the beginning of the starter phase until slaughter or from the beginning of grower phase until slaughter.

**13.** The composition for use according to any of claims 6 to 12, wherein the composition is a diet for poultry.

**14.** The composition for use according to any of claims 6 to 12, wherein the composition is an aqueous formulation.

**15.** Dimethyl sulfone and/or a dimethyl sulfone comprising composition for use in reducing moisture in litter wherein the dimethyl sulfone according to any of claims 1 to 5 and/or the dimethyl sulfone comprising composition according to any of claims 6 to 14 is administered to chronically heat stressed poultry.

**Patentansprüche**

**1.** Dimethylsulfon zur Verwendung bei der Behandlung von Hitzestress bei Geflügel durch orale Verabreichung, wobei das Dimethylsulfon an chronisch hitzegestresstes Geflügel verabreicht wird.

**2.** Dimethylsulfon zur Verwendung

gemäß Anspruch 1, wobei das Geflügel gegenüber einer Temperatur
von mehr als 27 °C, vorzugsweise gegenüber einer Temperatur von wenigstens 30 °C, exponiert ist.

**3.** Dimethylsulfon zur Verwendung

gemäß Anspruch 1 oder 2, wobei das Geflügel gegenüber einer Temperatur von mehr als 27 °C, vorzugsweise gegenüber einer
Temperatur von wenigstens 30 °C, für wenigstens 5 Stunden pro Tag exponiert ist.

**4.** Dimethylsulfon zur Verwendung

gemäß einem der Ansprüche 1 bis 3, wobei das Geflügel gegenüber einer
relativen Luftfeuchtigkeit von im Mittel wenigstens 40 % exponiert ist.

**5.** Dimethylsulfon zur Verwendung
gemäß einem der Ansprüche 1 bis 4, wobei das Dimethylsulfon an Geflügel in der Starter-, Mast-und/oder Endmastphase verabreicht wird.

**6.** Zusammensetzung umfassend Dimethylsulfon zur Verwendung bei der Behandlung von Hitzestress bei Geflügel durch orale Verabreichung, wobei die Zusammensetzung wenigstens 0,05 Gew.-% Dimethylsulfon bezogen auf das Gesamtgewicht der Zusammensetzung umfasst und an chronisch hitzegestresstes Geflügel verabreicht wird.

**7.** Zusammensetzung zur Verwendung

gemäß Anspruch 6, wobei die Zusammensetzung Dimethylsulfon
in einer Konzentration von 0,05 bis 0,3 Gew.-% bezogen auf das Gesamtgewicht der flüssigen und/oder festen Matrix der Zusammensetzung umfasst.

**8.** Zusammensetzung zur Verwendung

gemäß Anspruch 6 oder 7, wobei die Zusammensetzung an Geflügel verabreicht wird,
das gegenüber einer Temperatur von mehr als 27 °C, vorzugsweise gegenüber einer Temperatur von wenigstens 30 °C, exponiert ist.

**9.** Zusammensetzung zur Verwendung

gemäß einem der Ansprüche 6 bis 8, wobei die Zusammensetzung an
Geflügel verabreicht wird, das gegenüber einer Temperatur von mehr als 27 °C, vorzugsweise gegenüber einer
Temperatur von wenigstens 30 °C, für wenigstens 5 Stunden pro Tag exponiert ist.

**10.** Zusammensetzung zur Verwendung
gemäß einem der Ansprüche 6 bis 9, wobei die Zusammensetzung an Geflügel verabreicht wird, das gegenüber einer relativen Luftfeuchtigkeit von im Mittel wenigstens 40 % exponiert ist.

**11.** Zusammensetzung zur Verwendung

gemäß einem der Ansprüche 6 bis 10, wobei die Zusammensetzung an Geflügel in der Starter-, Mast- und/oder Endmastphase verabreicht wird.

**12.** Zusammensetzung zur Verwendung

gemäß einem der Ansprüche 6 bis 11, wobei die Zusammensetzung an Geflügel beginnend mit dem Beginn der Starterphase bis zu der Schlachtung oder mit dem Beginn der Mastphase bis zu der Schlachtung verabreicht wird.

**13.** Zusammensetzung zur Verwendung
gemäß einem der Ansprüche 6 bis 12, wobei die Zusammensetzung eine Nahrung für Geflügel ist.

**14.** Zusammensetzung zur Verwendung
gemäß einem der Ansprüche 6 bis 12, wobei die Zusammensetzung eine wässrige Formulierung ist.

**15.** Dimethylsulfon und/oder Dimethylsulfon-umfassende Zusammensetzung zur Verwendung zum Verringern von Feuchtigkeit in Einstreu, wobei das Dimethylsulfon gemäß einem der Ansprüche 1 bis 5 und/oder die Dimethylsulfon-umfassende Zusammensetzung gemäß einem der Ansprüche 6 bis 14 an chronisch hitzegestresstes Geflügel verabreicht wird.

## Revendications

**1.** Diméthylsulfone pour une utilisation dans le traitement du stress thermique chez de la volaille par administration orale, la diméthylsulfone étant administrée à de la volaille souffrant de manière chronique de stress thermique.

**2.** Diméthylsulfone pour une utilisation selon la revendication 1, la volaille étant exposée à une température de plus de 27 °C, préférablement à une température d'au moins 30 °C.

**3.** Diméthylsulfone pour une utilisation selon la revendication 1 ou 2, la volaille étant exposée à une température de plus de 27 °C, préférablement à une température d'au moins 30 °C, pendant au moins 5 heures par jour.

**4.** Diméthylsulfone pour une utilisation selon l'une quelconque des revendications 1 à 3, la volaille étant exposée à une humidité relative de l'air d'au moins 40 % en moyenne.

**5.** Diméthylsulfone pour une utilisation selon l'une quelconque des revendications 1 à 4, la diméthylsulfone étant administrée à de la volaille dans la phase de démarrage, de croissance et/ou de finition.

**6.** Composition comprenant de la diméthylsulfone pour une utilisation dans le traitement du stress thermique chez de la volaille par administration orale, la composition comprenant au moins 0,05 % en poids de diméthylsulfone, sur la base du poids total de la composition, et étant administrée à de la volaille souffrant de manière chronique de stress thermique.

**7.** Composition pour une utilisation selon la revendication 6, la composition comprenant de la diméthylsulfone en une concentration de 0,05 à 0,3 % en poids, sur la base du poids total de la matrice liquide et/ou solide de la composition.

**8.** Composition pour une utilisation selon la revendication 6 ou 7, la composition étant administrée à de la volaille qui est exposée à une température de plus de 27 °C, préférablement à une température d'au moins 30 °C.

**9.** Composition pour une utilisation selon l'une quelconque des revendications 6 à 8, la composition étant administrée à la volaille qui est exposée à une température de plus de 27 °C, préférablement à une température d'au moins 30 °C, pendant au moins 5 heures par jour.

**10.** Composition pour une utilisation selon l'une quelconque des revendications 6 à 9, la composition étant administrée

à de la volaille qui est exposée à une humidité relative de l'air d'au moins 40 % en moyenne.

11. Composition pour une utilisation selon l'une quelconque des revendications 6 à 10, la composition étant administrée à la de la volaille dans la phase de démarrage, de croissance et/ou de finition.

12. Composition pour une utilisation selon l'une quelconque des revendications 6 à 11, la composition étant administrée à de la volaille en démarrant par le début de la phase de démarrage jusqu'à l'abattage ou par le début de la phase de croissance jusqu'à l'abattage.

13. Composition pour une utilisation selon l'une quelconque des revendications 6 à 12, la composition étant un régime pour volaille.

14. Composition pour une utilisation selon l'une quelconque des revendications 6 à 12, la composition étant une formulation aqueuse.

15. Diméthylsulfone et/ou composition comprenant de la diméthylsulfone pour une utilisation dans la réduction de l'humidité dans la litière, la diméthylsulfone selon l'une quelconque des revendications 1 à 5 et/ou la composition comprenant de la diméthylsulfone selon l'une quelconque des revendications 6 à 14 étant administrée à la volaille souffrant de manière chronique de stress thermique.

Fig. 1:

Fig. 2:

Fig. 3:

Fig. 4:

Fig. 5:

Fig. 6:

Fig. 7:

Fig. 8:

Fig. 9:

Fig. 10:

Fig. 11:

Fig. 12:

Fig. 13:

Fig. 14:

Fig. 15:

Fig. 16:

Fig. 17:

Fig. 18:

**Litter moisture (in percent)**

□ T1: Basal diet (control)   ▨ T2: Basal diet + DSM (0.05%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. S. ABDUL RASHEED et al.** Dietary methylsulfonylmethane supplementation and oxidative stress in broiler chickens. *Poultry Science,* 22 January 2020, vol. 99 (2), 914-925